(19) Europäisches Patentamt
European Patent Office
Office européen des brevets

(11) Numéro de publication: **0 403 524 B1**

(12) # FASCICULE DE BREVET EUROPEEN

(45) Date de publication de fascicule du brevet: **10.02.93**

(51) Int. Cl.$^5$: **C07F 13/00**, C07B 59/00, A61K 43/00, A61K 49/00

(21) Numéro de dépôt: **89903166.0**

(22) Date de dépôt: **08.03.89**

(86) Numéro de dépôt internationale :
**PCT/FR89/00094**

(87) Numéro de publication internationale :
**WO 89/08657 (21.09.89 89/23)**

(54) **PREPARATION DE COMPLEXES NITRURO UTILISABLES COMME PRODUITS RADIOPHARMACEUTIOUES.**

(30) Priorité: **09.03.88 FR 8803044**
**25.11.88 FR 8815414**

(43) Date de publication de la demande:
**27.12.90 Bulletin 90/52**

(45) Mention de la délivrance du brevet:
**10.02.93 Bulletin 93/06**

(84) Etats contractants désignés:
**AT BE CH DE FR GB IT LI LU NL SE**

(56) Documents cités:
**WO-A-85/03063**

**Chemical Abstracts, vo. 95, No. 2, 13 July 1981 (Columbus, Ohio, US), L. Kaden et al.: "Nitrido complexes of technetium (V)", page 716, abstract No. 17322r, & Isotopenpraxis 1981, 17(4), 174-5, see abstract**

(73) Titulaire: **CIS BIO INTERNATIONAL**
**RN 306**
**F-91000 Saclay(FR)**

(72) Inventeur: **PASOUALINI, Roberto**
**223, avenue Victor-Hugo**
**F-92140 Clamart(FR)**
Inventeur: **MAGON, Luciano**
**Via Pietro Selvatico, 86**
**I-35100 Padova(IT)**
Inventeur: **BARDY, André**
**48, Les Genêts**
**F-91420 Morangis(FR)**
Inventeur: **DUATTI, Adriano**
**Via Galvana, 34**
**I-44040 Chiesval del Fosso, Ferrara(IT)**
Inventeur: **MARCHI, Andrea**
**Via Arianuova, 75**
**I-44100 Ferrara(IT)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

Chemical Abstracts, vol. 102, No. 24, 17 June 1985 (Columbus, Ohio, US), U. Abram et al.: "Lipophilic technetium complexes. III. Chelate complexes of tehcnetium (V) contianing the technetium-nitrido core", see page 694, abstract No. 214114p, & Inorg. Chem. Acta 1985, 109(1), L9-L11

(74) Mandataire: **Des Termes, Monique et al**
**Société Brevatome 25, rue de Ponthieu**
**F-75008 Paris(FR)**

## Description

La présente invention a pour objet un procédé de préparation de complexes d'un métal de transition choisi parmi $^{99m}Tc$, $^{186}Re$ et $^{188}Re$, utilisables comme produits radiopharmaceutiques ou pour la synthèse de nouveaux produits radiopharmaceutiques.

De façon plus précise, elle concerne la préparation de complexes nitruro d'un métal de transition M choisi parmi $^{99m}Tc$, $^{186}Re$ et $^{188}Re$, comportant une partie M≡N, dans laquelle M représente $^{99m}Tc$, $^{186}Re$ ou $^{188}Re$.

Ces complexes de métaux de transition radioactifs sont utilisables comme produits radiopharmaceutiques pour le diagnostic ou la thérapie.

Les complexes de technétium 99-m sont plutôt utilisées pour le diagnostic alors que les complexes complexes de rhénium 186 ou 188 sont plutôt utilisés pour la thérapie.

Les produits radiopharmaceutiques utilisant le radionucléide $^{99m}Tc$ sont des composés fréquemment utilisés en médecine nucléaire pour le diagnostic en raison des caractéristiques physiques et chimiques de ce radionucléide.

En effet, celui-ci donne uniquement une émission gamma, présente une énergie gamma optimale pour la détection externe (140 Kev) et une courte demi-vie physique (6,02h), ce qui permet de ne délivrer qu'une faible dose d'irradiation au patient. Par ailleurs, le coût de ce radioélément est peu élevé et il est disponible commercialement. Enfin, la richesse de la chimie du technétium permet d'obtenir une grande variété de produits radiopharmaceutiques.

En effet, comme il est indiqué par E. DEUTSCH et al. dans : Progr. Inorg. Chem. (Australia), vol.30, PP 76-106, 1983, le technétium peut former des complexes très variés avec de nombreux ligands, à des états d'oxydation allant de VII à -I et des nombres de coordination allant de 4 à 9.

Parmi les nombreux complexes du tableau I des pages 79-83 de ce document, on trouve des complexes de 99-Tc à l'état d'oxydation V, répondant aux formules :

$TcN\ Cl_2\ (PPh_3)_2$
$TcN\ Cl_2\ (PPh_3)_3$
$TcN\ Cl_2\ (PMe_2Ph)_3$
$TcN\ (S_2CNEt_2)_2$

Ces complexes ont été préparés par Kaden et al. selon le procédé décrit dans Isotopenpraxis, 1981, 17(4), pp.174-5, en vue d'être utilisés comme catalyseur dans la réaction complexe de réduction de l'azote élémentaire en ammoniac, découverte par Volpin et Shur. Ce procédé consiste à faire réagir du pertechnétate de $^{99}Tc$ avec du chlorhydrate d'hydrazine et la triphényl phosphine.

Pour cette préparation, on utilise 800 mg de $NH_4{}^{99}TcO_4{}^-$ et un rapport molaire $TcO_4{}^-/NH_2NH_2$, 2HCl de 0,46, c'est-à-dire une quantité de $^{99}Tc$ très supérieure à celle que l'on peut utiliser pour la préparation de produits radiopharmaceutiques où les quantités mises en jeu représentent environ 10 mCi de $^{99m}Tc$, soit $8.10^{-6}$ mg de $^{99m}Tc$.

En effet, la source de $^{99m}Tc$ est le générateur $^{99}Mo$-$^{99m}Tc$ qui fournit généralement une solution aqueuse de NaCl, 0,154M pure ou contenant des agents stabilisants, dans laquelle est présent l'ion pertechnétate ($^{99m}TcO_4^-$ + $^{99}TcO_4^-$ ) à des concentrations totales pouvant aller de $10^{-7}$ à $10^{-9}$ mol/l.

Aussi, le procédé de préparation décrit par Kaden et al. qui correspond à une synthèse macroscopique, ne peut être extrapolé à la synthèse de complexes nitruro de $^{99m}Tc$ à l'échelle microscopique où les rapports molaires entre $^{99m}Tc$ et les réactifs sont obligatoirement très inférieurs. De plus, la technique opératoire décrite par Kaden et al. qui nécessite une séparation par distillation, ne convient pas à la préparation de produits radiopharmaceutiques et est impraticable en milieu hospitalier, c'est-à-dire là où doivent être préparés les produits radiopharmaceutiques au moment de leur utilisation.

On connaît d'ailleurs un procédé de préparation de produits radiopharmaceutiques à base de complexes de technétium - 99m comportant une partie $(Tc{\equiv}N)^{2+}$ qui fait appel à une technique totalement différente. En effet, selon ce procédé qui est décrit par J. Baldas et Col. dans J. Chem. Soc. Dalton Trans. 1981, pp. 1798-1801 ; dans Int. J. Appl. Radiat. Isot. 36 (1985), pp.133-139 et dans la demande de brevet internationale WO85/03063, on prépare tout d'abord un composé de formule $R^+[^{99m}Tc{\equiv}NX_4]$ dans laquelle $R^+$ est un cation tel que la sodium ou l'ammonium et X représente un atome d'halogène tel que Cl ou Br, et on fait réagir ensuite ce composé avec un ligand approprié pour obtenir le complexe de $^{99m}Tc$ utilisable comme produit radiopharmaceutique.

Le complexe $R^+[^{99m}Tc{\equiv}NX_4]^-$ est intéressant car il est très stable à l'hydrolyse et peut être utilisé sans modification de la partie Tc≡N pour des réactions de substitution avec d'autres ligands, ce qui permet

d'obtenir une grande diversité de complexes de technétium.

Le procédé connu actuellement pour préparer le composé intermédiaire $R^+[^{99m}TCNX_4]^-$ consiste à faire réagir un pertechnétate tel que le pertechnétate de sodium avec de l'azoture de sodium et un hydracide halogéné tel que l'acide chlorhydrique.

Pour effectuer cette réaction, on évapore à sec une solution de pertechnétate de sodium (Tc-99m) en utilisant un évaporateur rotatif, puis on ajoute au résidu sec de l'azoture de sodium et de l'acide chlorhydrique concentré. On porte au reflux pendant environ 5 min pour compléter la réduction et détruire l'excès d'azoture et on évapore de nouveau à sec en utilisant un évaporateur rotatif. On obtient ainsi un résidu contenant le composé $R^+(^{99m}Tc{\equiv}NCl_4)^-$.

Ce procédé est difficilement applicable à la fabrication de trousses à usage médical car il est long et comprend au moins trois étapes dans lesquelles on utilise deux fois un évaporateur rotatif, ce qui n'est pas facile à mettre en oeuvre dans un service hospitalier de médecine nucléaire. De plus, ce procédé est difficilement utilisable pour la fabrication de trousses à usage médical car la stérilité et l'apyrogénéité des solutions sont difficilement contrôlables tout au long des opérations. De ce fait, le produit finalement obtenu doit être stérilisé après marquage par le Tc-99m, soit par passage sur une membrane stérilisante, soit par stérilisation à la chaleur, et il doit subir un contrôle d'absence de pyrogène avant l'injection sur l'homme.

Dans le cas de la préparation de complexes utilisables pour la thérapie, il est important d'utiliser un procédé de préparation conduisant à un rendement élevé en produits désirés.

Or, dans le cas du produit mis au point par Baldas ou Griffith (Coord. Chem. Rev. vol 8, 1972, pp.369-396) pour la préparation de complexes nitruro de technétium, on ne peut obtenir des rendements élevés en complexes.

Il en est de même avec les procédés généralement utilisés pour préparer des produits radiopharmaceutiques à base de rhénium.

La présente invention a précisément pour objet un procédé de préparation de complexes nitruro d'un métal de transition choisi parmi $^{99m}Tc$, $^{186}Re$ et $^{188}Re$, utilisables comme produits radiopharmaceutiques, qui pallie les inconvénients du procédé décrit ci-dessus.

Le procédé, selon l'invention, de préparation d'un produit radiopharmaceutique comprenant un complexe nitruro d'un métal de transition choisi parmi $^{99m}Tc$, $^{186}Re$ et $^{188}Re$, comportant une partie M≡N avec M représentant le métal de transition choisi parmi $^{99m}Tc$, $^{186}Re$ et $^{188}Re$, se caractérisé en ce que l'on fait réagir un composé oxygéné $MO_4^-$ du métal de transition M avec un premier ligand choisi dans le groupe des phosphines et polyphosphines aliphatiques et aromatiques, substituées ou non substituées, et un second ligand azoté constitué soit par un azoture d'ammonium ou de métal pharmaceutiquement acceptable, soit par un composé azoté comportant un motif $>N-N<$ dans lequel les N sont reliés à des atomes d'hydrogène et/ou à des groupements organiques monovalents par l'intermédiaire d'un atome de carbone ou dans lequel l'un des N est relié à l'atome de carbone d'un groupe organique bivalent par l'intermédiaire d'une double liaison et l'autre N est relié à des atomes d'hydrogène et/ou à des groupements organiques monovalents par l'intermédiaire d'un atome de carbone.

Selon ce procédé, on peut obtenir facilement un complexe nitruro d'un métal de transition car il suffit de mélanger les réactifs décrits ci-dessus pour former le complexe nitruro.

Généralement, les premier et second ligands sont utilisés sous la forme de solutions alcoolique ou hydroalcoolique, et par simple addition de ces solutions au composé oxygéné du métal de transition, par exemple à du pertechnétate de sodium ou du perrhénate de sodium, on obtient directement un produit comportant le complexe nitruro recherché.

Ainsi, dans ce cas, il n'est pas nécessaire d'effectuer tout d'abord des étapes d'évaporation à sec des réactifs. Il n'est pas davantage nécessaire de stériliser le produit obtenu en fin de réaction car il suffit d'utiliser une solution stérile du composé oxygéné du métal de transition.

De plus, le procédé de l'invention permet l'obtention de rendements élevés, ce qui n'était pas le cas avec les procédés de l'art antérieur.

Les composés oxygénés de métaux de transition M utilisés dans l'invention sont des sels du type $MO_4M'$, avec M' représentant un métal alcalin ou l'ammonium.

Dans le procédé de l'invention, le premier ligand à base de phosphine agit comme agent réducteur du métal de transition et favorise la formation du coeur M≡N ainsi que la fixation quantitative du second ligand azoté. En effet, en l'absence du premier ligand, il est impossible d'obtenir par réaction du second ligand avec le composé oxygéné du métal de transition un complexe comportant une partie M≡N.

De préférence, selon l'invention, la réaction entre le composé oxygéné du métal de transition et le premier et le second ligands est effectuée en solution aqueuse.

Pour réaliser la réaction, on peut introduire aseptiquement le second ligand azoté et la phosphine, de préférence en solution aqueuse, dans un récipient, puis ajouter la quantité requise de composé oxygéné de

métal de transition, par exemple de pertechnétate de technétium 99m, après avoir ajusté le pH à une valeur appropriée par addition d'acide ou de base. On peut ensuite effectuer la réaction à la température ambiante ou à une température supérieure allant de 50 à 100 °C. La température et le pH utilisés dépendent en particulier du second ligand azoté. Généralement on opère à des pH inférieurs à 4.

Généralement, on opère avec des rapports molaires composé oxygéné du métal de transition /1er ligand azoté de $10^{-9}$ à $10^{-4}$.

Le produit obtenu par ce procédé peut être utilisé tel quel comme produit radiopharmaceutique pour la thérapie ou le diagnostic.

Il peut aussi servir de produit intermédiaire pour la fabrication d'autres complexes nitruro utilisables comme produits radiopharmaceutiques pour le diagnostic ou la thérapie. Dans ce cas, on échange les ligands du complexe nitruro de technétium obtenu précédemment par un troisième ligand organique à groupement nucléophile ayant par exemple un tropisme meilleur pour certains organes du corps humain ou encore par un anticorps monoclonal ou un fragment d'anticorps.

Cette réaction d'échange peut être effectuée simultanément, de préférence en solution aqueuse, lors de la formation du complexe nitruro en faisant réagir ensemble le composé oxygéné du métal de transition, le premier ligand, le second ligand azoté et le troisième ligand organique à groupement nucléophile, l'anticorps monoclonal ou le fragment d'anticorps. On peut aussi effectuer cette réaction en deux étapes, soit une première étape, réalisée de préférence en solution aqueuse, dans laquelle on fait réagir le composé oxygéné du métal de transition avec le premier et le second ligands, puis une deuxième étape, réalisée de préférence aussi en solution aqueuse, dans laquelle on fait réagir le produit obtenu à la suite de la première étape avec le troisième ligand, l'anticorps monoclonal ou le fragment d'anticorps.

On peut toutefois réaliser cette réaction d'échange en solution alcoolique ou hydroalcoolique ; il est aussi possible d'effectuer la première étape et la deuxième étape dans des solutions différentes par exemple la première étape en solution aqueuse et la deuxième étape en solution alcoolique ou hydroalcoolique ou l'inverse.

Les ligands organiques à groupement nucléophile utilisés pour cette réaction d'échange peuvent être très divers. A titre d'exemple, on peut utiliser des amines, des thiols, des thioéthers, des oximes, des phosphines et des ligands polyfonctionnels du type polyaminopolythiol.

Lorsque la réaction est effectuée avec un anticorps monoclonal ou un fragment d'anticorps, on peut préparer de cette façon un anticorps marqué par un métal de transition. Pour cette réaction, l'anticorps monoclonal ou le fragment d'anticorps utilisé peut être activé, par exemple par prétraitement avec le 2-aminoéthane-thiol ou un agent réducteur tel que le dithiothréitol, pour convertir les liaisons disulfure en groupe sulfhydryle.

On peut utiliser de nombreux types d'anticorps, en particulier ceux capables de se lier à la partie M≡N par des atomes de soufre. A titre d'exemples de tels anticorps, on peut citer les anticorps anti-ACE (anti-antigène carcinoembryonnaire), les anticorps anti-carcinome ovarien (OC125), les anticorps anti-colorectal, anti-fibrine, anti-myosine.

L'anticorps ou le fragment d'anticorps marqué obtenu est très intéressant, par exemple pour la détection des tumeurs. En effet, après réaction avec le complexe de métal de transition, l'anticorps monoclonal ou le fragment d'anticorps est lié à l'élément de transition tel que le technétium 99m, mais il peut réagir avec les antigènes correspondants. Ainsi, la spécificité de l'anticorps est maintenue et l'anticorps marqué est stable. Aussi, on peut utiliser cet anticorps marqué pour la détection de tumeurs car il se dirigera naturellement vers l'antigène correspondant et permettra la visualisation des tumeurs.

Dans le procédé de l'invention, le choix des ligands utilisés est important car il conditionne les propriétés du produit obtenu.

Le premier ligand qui permet d'obtenir la formation d'un complexe nitruro, est un ligand organique à atome de phosphore donneur d'électrons choisi parmi les phosphines et les polyphosphines aliphatiques et aromatiques, substituées ou non substituées.

Les phosphines utilisables peuvent répondre à la formule :

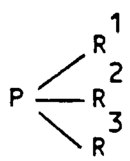

dans laquelle $R^1$, $R^2$ et $R^3$ qui peuvent être identiques ou différents, représentent un atome d'hydrogène, un

radical alkyle, un radical aryle, un radical alcoxy ou un radical alkyle ou aryle substitué par un groupement choisi parmi les radicaux amino, amido, cyano et sulfonate.

A titre d'exemple de phosphines de ce type, on peut citer la triphénylphosphine, la diéthylphénylphosphine, la triéthylphosphine, la triméthylphosphine, la tris(cyanoéthyl-2) phosphine et la triphénylphosphine sulfonée. Généralement, on préfère utiliser des triarylphosphines comme la triphénylphosphine, car celles-ci sont moins oxydables que les trialkylphosphines.

Les polyphosphines susceptibles d'être utilisées dans l'invention peuvent répondre aux formules :

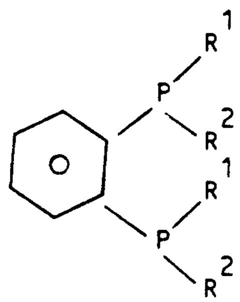

ou

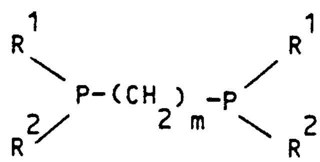

dans lesquelles $R^1$ et $R^2$ qui peuvent être identiques ou différents, représentent un atome d'hydrogène, un radical alkyle, un radical aryle, un radical alcoxy ou un radical alkyle ou aryle substitué par un groupement choisi parmi les radicaux amino et amido, et m est un nombre entier allant de 1 à 4.

A titre d'exemples de telles polyphosphines, on peut citer le bis(diméthyl-1,2-phosphino)éthane et le bis(diphényl-1,2-phosphino)éthane.

Comme on l'a vu précédemment, le second ligand peut être un azoture d'un métal pharmaceutiquement acceptable ou d'ammonium, ou un composé azoté comportant le motif >N-N< .

Les azotures de métaux pharmaceutiquement acceptables peuvent être en particulier les azotures de métaux alcalins, par exemple l'azoture de sodium.

Les composés azotés comportant le motif >N-N< peuvent répondre à la formule :

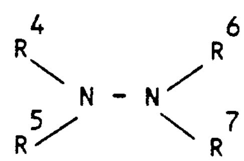

dans laquelle $R^4$, $R^5$, $R^6$ et $R^7$ qui peuvent être identiques ou différents, représentent un atome d'hydrogène ; un radical alkyle ; un radical aryle ; un radical alcoxy ; un radical alkyle substitué par au moins un groupement choisi parmi les radicaux hydroxy, carboxy, amino, amido et mercapto ; un radical aryle substitué par au moins un groupement choisi parmi les atomes d'halogène et les radicaux alcoxy, hydroxy, amino, mercapto et amino substitué par au moins un radical alkyle ; un radical répondant aux formules :

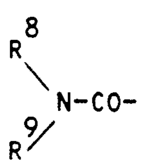 , 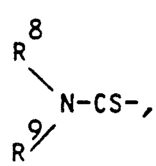 ou 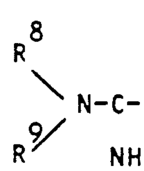

dans lesquelles $R^8$ et $R^9$ qui peuvent être identiques ou différents, représentent un atome d'hydrogène, un radical alkyle, ou un radical amino ; un radical de formule :

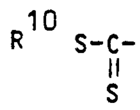

dans laquelle $R^{10}$ représente un atome d'hydrogène, un radical alkyle ou un radical aryle ; un radical de formule $R^{11}$ -CO- avec $R^{11}$ représentant un radical alkyle, un radical alcoxy, un radical aryle non substitué ou substitué par au moins un groupement choisi parmi les atomes d'halogène et les radicaux hydroxy, ou un radical dérivé d'un hétérocycle non substitué ou substitué par au moins un groupement choisi parmi les atomes d'halogène et les radicaux hydroxy ; ou dans laquelle $R^4$ et $R^5$ peuvent former ensemble un radical bivalent de formule :

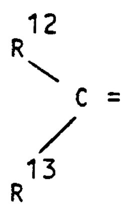

dans laquelle $R^{12}$ représente $-CH_2-NH_2$, un radical aryle non substitué ou substitué par au moins un groupement choisi parmi les atomes d'halogène et les radicaux hydroxy, alcoxy, amino, mercapto et amino substitué par au moins un radical alkyle, ou un radical dérivé d'un hétérocycle non substitué ou substitué par un ou plusieurs groupements choisis parmi les atomes d'halogène et les radicaux hydroxy, alcoxy, amino, mercapto et amino substitué par au moins un radical alkyle et, $R^{13}$ représente un atome d'hydrogène, un radical alkyle ou un radical alkyle substitué par au moins un groupement choisi parmi les radicaux hydroxy, carboxy, amino, amido et mercapto ; et $R^6$ et $R^7$ ont la signification donnée ci-dessus.

Ces composés azotés peuvent appartenir en particulier :

- au groupe de l'hydrazine et de ses dérivés comme par exemple les alkylhydrazines, les semicarbazides, les hydrazides, les thiosemicarbazides, les carbohydrazides, les thiocarbohydrazides, l'acéthydrazide, les hydrazinecarboxylates et les aminoguanidines,
- au groupe de l'acide dithiocarbazique et de ses dérivés, et
- au groupe des produits obtenus par condensation des composés décrits ci-dessus avec des cétones ou des aldéhydes aliphatiques ou aromatiques.

Ainsi, le second ligand azoté peut être l'acide dithiocarbazique ou un dérivé de celui-ci répondant à la formule :

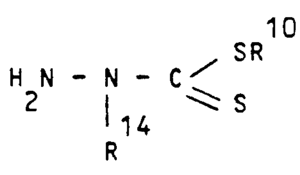

dans laquelle $R^{10}$ représente un atome d'hydrogène, un radical alkyle ou un radical aryle, et $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alcoxy, hydroxy, amino, mercapto et amino substitué par au moins un radical alkyle.

Il peut également être un produit de condensation obtenu par réaction de l'acide dithiocarbazique avec une cétone ou un aldéhyde aliphatique de formule $R^{15}$-CO-$R^{16}$. Dans ce cas, il répond à la formule :

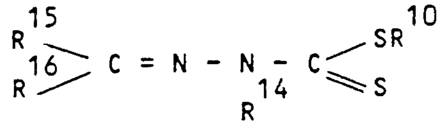

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alcoxy, hydroxy, amino, mercapto et amino substitué par au moins un radical alkyle ; et $R^{15}$ et $R^{16}$ qui peuvent être identiques ou différents représentent un atome d'hydrogène, un radical alkyle ou un radical alkyle substitué par au moins un groupement choisi parmi les radicaux hydroxy, carboxy, amino, amido et mercapto.

Le dérivé d'acide dithiocarbazique utilisé comme second ligand peut aussi être le produit de condensation de l'acide dithiocarbazique avec une cétone ou un aldéhyde aromatique. Dans ce cas, le dérivé répond à la formule :

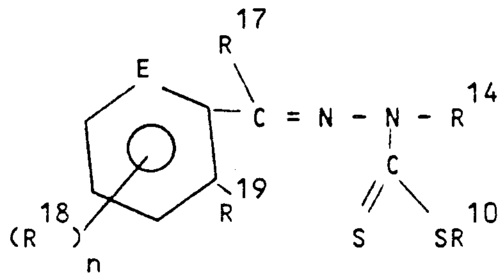

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle ; $R^{17}$ représente un atome d'hydrogène, un radical alkyle, un radical alkyle substitué par au moins un groupement choisi parmi les radicaux hydroxy, carboxy, amino, amido et mercapto, $R^{18}$ représente un atome d'hydrogène, un atome d'halogène, un radical alcoxy, un radical amino, ou un radical amino substitué par au moins un groupement alkyle, $R^{19}$ représente un atome d'hydrogène, un radical hydroxy ou un radical mercapto, E représente un atome de carbone ou un atome d'azote, et n est un nombre entier allant de 1 à 4, ou dans laquelle n est égal à 2 et les deux $R^{18}$ sont voisins et forment ensemble un cycle aromatique.

On peut également utiliser comme second ligand le produit obtenu par condensation de l'acide dithiocarbazique avec une cétone comportant un hétérocycle à 5 maillons. Dans ce cas, le second ligand répond à la formule :

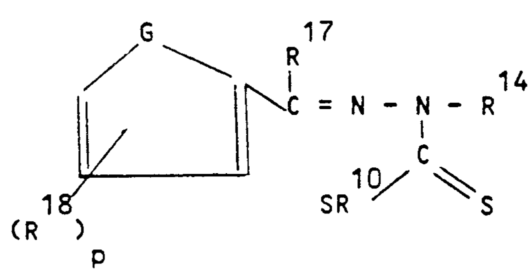

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux

alcoxy, hydroxy, amino, mercapto et amino substitué par au moins un radical alkyle ; $R^{17}$ représente un atome d'hydrogène, un radical alkyle, un radical alkyle substitué par au moins un groupement choisi parmi les radicaux hydroxy, carboxy, amino, amido et mercapto, $R^{18}$ représente un atome d'hydrogène, un atome d'halogène, un radical alcoxy, un radical amino, ou un radical amino substitué par au moins un groupement alkyle, G est S ou O, et p est 1, 2 ou 3.

Le second ligand peut encore être l'hydrazine ou un dérivé d'hydrazine répondant à la formule :

$R^{20}$-NH-NH-$R^{21}$

dans laquelle $R^{20}$ est un atome d'hydrogène ou un radical alkyle et $R^{21}$ est un atome d'hydrogène ou un radical choisi parmi les radicaux de formule :

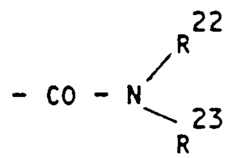

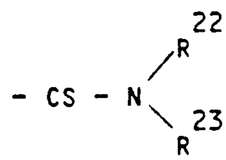

-CO $R^{24}$
-COO $R^{24}$
-CO-$R^{25}$

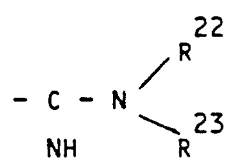

dans lesquelles $R^{22}$ et $R^{23}$ qui peuvent être identiques ou différents, représentent un atome d'hydrogène, un radical alkyle ou un radical amino, $R^{24}$ représente un radical alkyle et $R^{25}$ représente un radical aryle non substitué ou substitué par au moins un groupement choisi parmi les atomes d'halogène et les radicaux hydroxy ou un radical dérivé d'un hétérocycle non substitué ou substitué par au moins un groupement choisi parmi les atomes d'halogène et les radicaux hydroxy.

Les radicaux alkyle et alcoxy utilisés dans les ligands décrits ci-dessus peuvent être des radicaux linéaires ou ramifiés ; ils ont généralement de 1 à 3 atomes de carbone.

Les radicaux aryle sont des radicaux dérivés d'un noyau par élimination d'un atome d'hydrogène tels que les radicaux phényle et naphtyle. A titre d'exemple, les radicaux dérivés des noyaux hétérocycliques peuvent être des radicaux furfuryle, pyridyle, thiofurfuryle.

Les ligands décrits ci-dessus sont des produits du commerce ou peuvent être préparés par des procédés classiques.

Dans le procédé de l'invention où le métal de transition est le technétium ou le rhénium, les ligands azotés utilisés peuvent être monodentés, bidentés ou tridentés.

En effet, il semble que le complexe nitruro obtenu ait la structure pyramidale de base carrée suivante dans le cas du Tc :

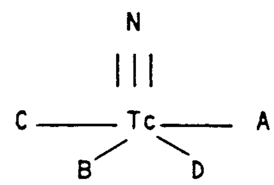

dans laquelle A représente le ligand à base de phosphine et B, C et D représentent le ou les ligands azotés.

Dans le cas d'un ligand azoté tridenté, tel que le S-méthyl-bêta-N(2-hydroxyphényl)méthylène-dithiocarbazate de formule :

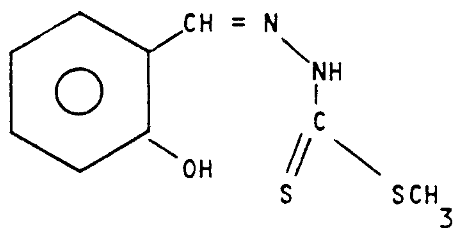

la position B est occupée par l'un des atomes d'azote du dithiocarbazate alors que les positions C et D sont occupées par les atomes $O^-$ et $S^-$ du dithiocarbazate ionisé. Le complexe a ainsi la structure suivante :

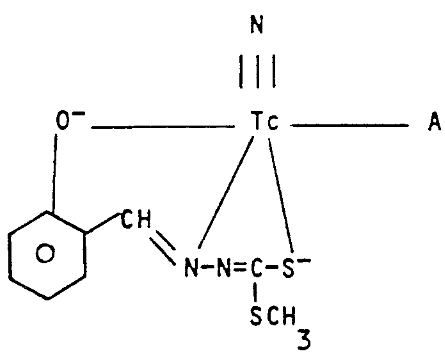

Ainsi, si l'on utilise un ligand azoté tridenté, on obtiendra un seul complexe. En revanche, lorsqu'on utilise des ligands azotés monodentés, on peut avoir différents types de complexes selon que le premier ligand du type phosphine occupe une ou plusieurs des positions A, B, C et D du complexe.

Lorsqu'on fait réagir le complexe obtenu à partir du premier et du second ligands avec un troisième ligand organique à groupement nucléophile, il est nécessaire que ce troisième ligand organique soit monodenté, bidenté ou tétradenté dans le cas où le métal de transition est Tc. En effet, dans ce cas, on peut obtenir le remplacement du premier et du second ligands par le troisième ligand tétradenté. Dans le cas de ligands mono ou bidentés, plusieurs complexes peuvent être préparés, mais on observe qu'il se produit généralement un réarrangement des complexes obtenus en faveur du complexe de technétium comportant surtout le troisième ligand.

Dans l'invention, le choix des ligands utilisés a une grande importance car il conditionne en particulier les propriétés du produit radiopharmaceutique obtenu. En effet, en choisissant un second ligand azoté ayant une affinité particulière pour certains organes avec du Tc-99m, convenant aux examens par scintigraphie, on peut préparer une composition radiopharmaceutique utilisable directement pour le diagnostic.

Dans ce cas, il est bien entendu nécessaire également que le premier et le second ligands ne soient pas toxiques et puissent être administrés à l'homme.

Aussi, l'invention a également pour objet une trousse pour la préparation d'un produit radiopharmaceutique comprenant un complexe nitruro de $^{99m}Tc$, $^{186}Re$ ou $^{188}Re$, qui comprend :

- un premier flacon contenant le premier ligand du type phosphine et un second flacon contenant le second ligand azoté.

Ainsi, on peut préparer directement à partir de cette trousse le produit radiopharmaceutique voulu, dans un service hospitalier de médecine nucléaire, en mélangeant le contenu des deux flacons et en lui ajoutant par exemple une solution de pertechnétate de métal alcalin ou d'ammonium. Le premier et le second ligands peuvent être présents respectivement dans les premier et second flacons sous forme liquide ou sous forme lyophylisée.

Dans certains cas, on peut aussi mélanger dans le même flacon le premier et le second ligands et ajouter au dernier moment la solution du composé oxygéné de métal de transition, par exemple de pertechnétate ou de perrhénate, pour préparer le produit radiopharmaceutique.

Comme on l'a vu précédemment, on peut aussi utiliser le complexe de métal de transition obtenu à partir du premier et du second ligands comme produit intermédiaire pour la préparation d'un autre complexe nitruro de métal de transition par réaction d'échange avec un troisième ligand, un anticorps monoclonal ou un fragment d'anticorps.

Le produit obtenu à la suite de cette réaction peut également être utilisé tel quel comme produit radiopharmaceutique soit pour le diagnostic, soit pour la thérapie. Dans ce cas, la trousse permettant la préparation du produit radiopharmaceutique peut comprendre un troisième flacon contenant le troisième ligand organique à groupement nucléophile, l'anticorps monoclonal ou le fragment d'anticorps.

D'autres caractéristiques et avantages de l'invention apparaîtront mieux à la lecture des exemples suivants donnés bien entendu à titre illustratif et non limitatif.

**EXEMPLE 1**

Dans un flacon type Pénicilline, on introduit 0,4 ml d'une solution contenant $2.10^{-2}$ mol/l (2,5 mg/ml) de S-méthyldithiocarbazate (second ligand) dans l'alcool éthylique, puis 0,2 ml d'une solution à $2.10^{-2}$ mol/l (5mg/ml) de triphénylphosphine (premier ligand) dans l'alcool éthylique, et 0,1 ml d'acide chlorhydrique 1N. On ajoute ensuite 0,5 à 1 ml d'une solution de pertechnétate de sodium (Tc-99m), ($10^{-9}$ - $10^{-11}$ mol de Tc), et on effectue la réaction à 80 ° C, pendant 30 minutes.

L'analyse chromatographique sur couche mince du produit obtenu montre qu'il s'agit d'un complexe nitruro de technétium comportant le coeur Tc≡N.

**EXEMPLES 2 à 13**

On suit le même mode opératoire que dans l'exemple 1 pour préparer à partir des ligands donnés dans le tableau 1 qui suit, des complexes nitruro de technétium en introduisant dans le flacon $5.10^{-3}$ à $1.10^{-2}$ mmol du second ligand, $4.10^{-3}$ mmol du premier ligand et 0,1 ml de HCl 1N, et en ajoutant ensuite 0,5 à 1ml de solution de pertechnétate de sodium.

L'analyse chromatographique des produits obtenus montre qu'il s'agit bien de complexes nitruro comportant la partie Tc≡N.

**EXEMPLE 14**

Dans un flacon type pénicilline, on introduit 0,4 ml d'une solution alcoolique du second ligand, constituée par une solution à 2,5 mg/ml ($1,1.10^{-2}$ mol/l) de S-méthyl-bêta-N(2-hydroxyphényl)méthylène dithiocarbazate dans l'alcool éthylique, 0,2ml d'une solution à 5 mg/l ($2.10^{-2}$ mol/l) de triphénylphosphine (premier ligand) dans l'alcool éthylique, et 0,1ml d'une solution d'acide chlorhydrique 1N.

On ajoute ensuite 0,5ml à 1ml d'une solution stérile de pertechnétate de sodium (technétium-99m) correspondant à une radioactivité allant de 18 M Bq à 3,7 GBq (0,5 à 100 mCi), et on chauffe le flacon à 80 ° C pendant 30 minutes.

L'analyse chromatographique sur couche mince en phase inverse Whatman KC 18 en utilisant comme solvant un mélange de méthanol, acétonitrile, tétrahydrofurane et acétate d'ammonium 0,5M (proportions 3:3:2:2) montre l'apparition d'un produit pur ayant un Rf de 0,35, et confirme la présence de la partie Tc≡N.

**EXEMPLES 15 à 22**

On répète le mode opératoire de l'exemple 14 avec les premiers et seconds ligands du tableau 2 en introduisant dans le flacon $1.10^{-2}$ à $3.10^{-3}$ mmole du second ligand, $4.10^{-3}$ mmole du premier ligand et 0,1 ml de HCl 1N, et en ajoutant ensuite 0,5 à 1 ml de solution de pertechnétate de sodium Tc-99m.

En fin d'opération, on soumet le produit obtenu à une chromatographie sur couche mince et on vérifie ainsi que le complexe comporte la partie Tc≡N et que la phosphine fait partie intégrante du complexe obtenu.

**EXEMPLE 23**

On suit le même mode opératoire que dans l'exemple 1 mais en utilisant comme second ligand le 4-méthyl-3-thiosemicarbazide de formule :

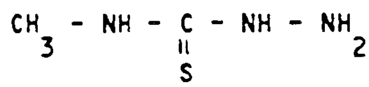

On vérifie ensuite par analyse chromatographique que le complexe obtenu est bien un complexe nitruro de technétium et qu'il comprend la triphénylphosphine.

**EXEMPLE 24**

Dans cet exemple, on suit le même mode opératoire que dans l'exemple 14 mais en utilisant comme second ligand l'aminoacétone semicarbazone de formule :

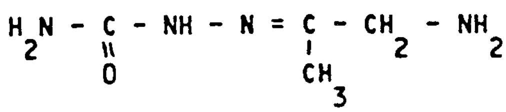

On vérifie ensuite par chromatographie sur couche mince que le complexe obtenu est bien un complexe nitruro de technétium.

**EXEMPLE 25**

Dans cet exemple, on introduit dans un flacon type pénicilline 1ml d'une solution à $5.10^{-3}$ mol/l de alpha-N-méthyl S-méthyl-bêta-N(2-hydroxyphényl)méthylène dithiocarbazate dans l'alcool éthylique, 0,2ml d'une solution à $6.10^{-3}$ mol/l de bis(diméthyl-1,2-phosphino)éthane de formule :

$(CH_3)_2P - CH_2 - CH_2 - P\ (CH_3)_2$

et 0,1ml d'acide chlorhydrique 1N. On ajoute ensuite 0,5ml d'une solution stérile de pertechnétate de sodium (Tc-99m) et on chauffe le flacon à 80 ° C pendant 30 minutes.

On obtient ainsi un complexe nitruro de technétium comportant le premier ligand à base de diphosphine.

**EXEMPLE 26**

On suit le même mode opératoire que dans l'exemple 25 mais en utilisant une solution à $5.10^{-3}$ mol/l de bis(diphényl-1,2-phosphino)éthane et une solution à $5.10^{-3}$ mol/l de alpha-N-méthyl S-méthyl-bêta-N(2-hydroxyphényl) méthylènedithiocarbazate.

On obtient également un complexe nitruro de technétium comportant le ligand diphosphine.

**EXEMPLE 27**

Dans cet exemple, on prépare un complexe de rhénium -186 en introduisant dans un flacon type pénicilline 7 mmole de triphénylphosphine et 2 mmole de alpha-N-méthyl S-méthyl-bêta-n(2-hydroxyphényl)méthylènedithiocarbazate en solution alcoolique et 5 mmoles d'acide chlorhydrique 1N. On introduit ensuite 1 mmole de perrhénate de sodium et on effectue la réaction à 40 ° C pendant 30 minutes.

On obtient ainsi un complexe nitruro de rhénium avec un rendement supérieur à 90%.

Dans les exemples qui suivent, on utilise les complexes nitruro de technétium obtenus dans les

exemples précédents pour former d'autres complexes de technétium utilisables comme produits de diagnostic.

**EXEMPLE 28**

Dans cet exemple, on utilise le produit obtenu dans l'exemple 14 pour préparer un autre complexe de technétium avec un troisième ligand constitué par du 1,1'-(1,2-éthane diyl-diimino)-bis-(2-méthyl-2-propane thiol) de formule :

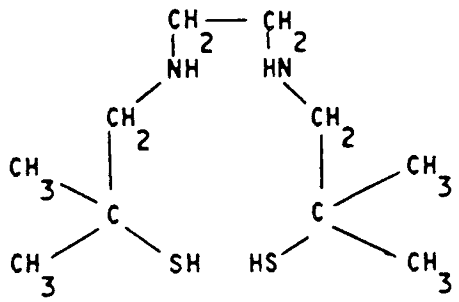

Au contenu du flacon obtenu dans l'exemple 14, on ajoute 0,2ml d'une solution à $4.10^{-2}$ mol/l de 1,1'-(1,2-éthane diyl-diimino)-bis-(2-méthyl-2-propane thiol) dans de l'éthanol.

On amène le pH à 9,5 par addition de 0,5ml d'une solution tampon carbonate/bicarbonate 0,5M, et on chauffe le flacon à 80 °C pendant 30 minutes.

On soumet le produit obtenu à une chromatographie sur couche mince en utilisant du silicagel et un solvant formé d'éthanol-chloroforme-benzène (2:2:1). Les emplacements des taches chromatographiques correspondent à un complexe nitruro de technétium comportant la partie Tc≡N.

**EXEMPLE COMPARATIF 1**

Dans cet exemple, on fait réagir une solution de 1,1'-(1,2-éthane diyl-diimino)-bis-(2-méthyl-2-propane thiol) avec du pertechnétate de sodium (Tc-99m) en présence de chlorure stanneux. On obtient ainsi un diaminodithiolate oxotechnétium que l'on analyse par chromatographie sur couche mince dans les mêmes conditions que le produit obtenu dans l'exemple 27.

L'emplacement des taches chromatographiques pour ce complexe de Tc contenant le coeur $(TcO)^{3+}$ est différent de celui obtenu dans l'exemple 26 avec le complexe nitruro.

**EXEMPLES 29 à 36**

On suit le même mode opératoire que dans l'exemple 28 pour préparer d'autres complexes nitruro de technétium à partir des produits obtenus dans les exemples 1, 2 et 16 à 21 en utilisant comme troisième ligand le 1,1'-(1,2-éthane diyl-diimino)-bis-(2-méthyl-2-propane thiol).

Dans tous les cas, on obtient un complexe nitruro de technétium comportant ce troisième ligand.

**EXEMPLE 37**

On suit le même mode opératoire que dans l'exemple 28 pour préparer un nouveau complexe de technétium à partir du produit obtenu dans l'exemple 1 mais en utilisant comme troisième ligand une solution à $6.10^{-2}$ mol/l de tétraazaundécane de formule :

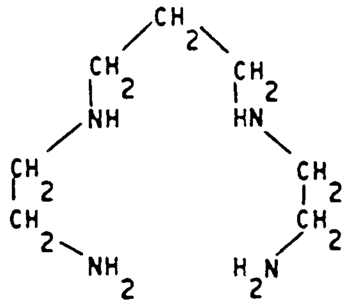

On obtient ainsi un nouveau complexe nitruro de technétium comportant comme ligand le tétraazaundécane.

La pureté du produit est testée par chromatographie sur couche mince en utilisant la cellulose et un solvant à base d'éthanol-acétate d'ammonium 0,15M (rapport 4:3).

**EXEMPLES 38 à 45**

Dans ces exemples, on suit le même mode opératoire que dans l'exemple 37 en ajoutant au produit obtenu dans les exemples 2, 14 et 16 à 21, 0,2ml d'une solution à $6.10^{-2}$ mol/l de tétraazaundécane.

On obtient dans tous les cas un complexe nitruro de technétium comportant le ligand tétraazaundécane.

**EXEMPLE 46**

Au contenu du flacon obtenu dans l'exemple 16, on ajoute 0,1ml d'une solution à $6.10^{-2}$ mol/l (13mg/ml) de dichlorhydrate de bis(1,2-diméthylphosphine)éthane (DMPE). On amène le pH à 10 par addition de 0,5ml d'une solution tampon bicarbonate/carbonate à 0,5 mol/l et on effectue la réaction à 80° C pendant 30 minutes.

On analyse le produit obtenu par chromatographie sur couche mince (cellulose ; solvant : éthanol-acétate d'ammonium 0,15M, 4:3). On constate ainsi qu'il s'agit d'un complexe nitruro de technétium comprenant le troisième ligand.

En effet, les taches chromatographiques obtenues avec ce complexe sont différentes de celles que l'on obtient avec les complexes connus de technétium ayant pour formule :

$^{99m}TcCl_2DMPE_2{}^+$ et $^{99m}TcO_2DMPE_2{}^+$.

**EXEMPLE 47 :** Préparation d'un complexe $^{99m}Tc{\equiv}N$ contenant de la 8-mercapto quinoléine.

a) - Préparation du produit intermédiaire.

Dans un flacon type pénicilline, on introduit 0,2 ml d'une solution contenant $7,7.10^{-2}$ mole/l (5mg/ml) d'azoture de sodium dans l'eau, puis 0,5 ml d'une solution contenant $5,2.10^{-3}$ mole/l (1mg/ml) de tris-(cyanoéthyl-2) phosphine dans l'eau.

On ajoute ensuite 0,5 à 5 ml d'une solution de pertechnétate de sodium ($^{99m}Tc$) et on effectue la réaction à 80° C pendant 30 minutes ou à 100° C pendant 15 minutes.

b) - Préparation du complexe final.

Au contenu du flacon obtenu dans l'étape a), ou à une fraction de celui-ci, on ajoute 0,3 ml d'une solution tampon carbonate-bicarbonate de sodium 0,5 mole/l à pH 9,0 et 0,4 ml d'une solution contenant $5.10^{-2}$ mole/l de chlorhydrate de 8-mercapto quinoléine (10 mg/ml) dans l'éthanol.

On effectue la réaction 15 minutes à 100° C, 30 minutes à 80° C ou pendant 60 minutes à température ambiante.

La pureté radiochimique du complexe obtenu est testée en effectuant une chromatographie sur couche mince en utilisant un gel de silice et un mélange d'éthanol, chloroforme, toluène, et acéte d'ammonium (0,5M) dans les proportions 6/3/3/1.

Le complexe obtenu a un Rf de 0,95 alors que le $^{99m}TcO_4^-$ à un Rf de 0,5.

La pureté radiochimique est supérieure ou égale à 95%.

**EXEMPLE 48 :** Préparation d'anticorps marqué avec le complexe intermédiaire Tc≡N.

a) - Préparation du produit intermédiaire

Dans un flacon type pénicilline on introduit 0,4 ml d'une solution contenant $2.10^{-2}$ mole/l (2,7mg/ml) de S-méthyl, N-méthyl dithiocarbazate dans l'eau, puis 0,5 ml d'une solution contenant $5,2.10^{-3}$ mole/l de tris-(cyanoéthyl-2) phosphine dans l'eau et 0,1 ml d'acide chlorhydrique 1N.

On ajoute ensuite 0,5 à 5 ml d'une solution de pertechnétate de sodium ($^{99m}Tc$) et on effectue la réaction à 80 °C pendant 30 minutes ou à 100 °C pendant 15 minutes.

b) - Préparation de l'anticorps marqué.

Au contenu du flacon obtenu dans l'étape a) amené à pH 7, ou à une fraction de celui-ci, on ajoute 1 mg d'anticorps monoclonal entier anti-ACE (anti- antigène carcinoembryonnaire) prétraité par la -mercapto éthanolamine pour activer les groupements sulfhydryle (anticorps entier activé) dans une solution tampon phosphate 0,1M à pH 7,0.

La réaction a lieu pendant 30 minutes à 35 °C.

La pureté radiochimique est testée en effectuant une chromatographie par filtration sur gel sur colonne TSK type G 3000 SW (0,75x30 cm) par tampon phosphate 0,1M pH 7,0 au débit de 1 ml/minute. La radioactivité et l'absorbance de l'échantillon sont enregistrées simultanément.

95% de la radioactivité est éluée entre 7 et 7,8 ml et la quantité de $^{99m}TcO_4^-$ détectable, à 12 ml est inférieure à 5%.

**EXEMPLES 49 à 55**

Dans ces exemples on teste les propriétés des complexes obtenus dans les exemples 2, 14, 17, 21, 28, 37 et 47 en déterminant leur biodistribution chez des rats mâles de la race Sprague Dawley pesant 200 + 20g. Dans ce cas on injecte aux rats, anesthésiés avec du pentobarbital, une dose de rayonnement de 3,7 KBq à 10 KBq (1 à 2,7 µCi).

Les animaux sont sacrifiés 5 minutes après l'injection du produit. Les organes sont prélevés, lavés, et leur radioactivité mesurée au moyen d'un compteur à scintillation.

Les résultats obtenus sont donnés dans le tableau 3 annexé. Ils sont exprimés en pourcentage de la radioactivité injectée retrouvée dans l'organe après prélèvement et comptage. Le valeurs données dans chaque case du tableau représentent la valeur moyenne de trois expériences.

TABLEAU 1

| EX | 1er ligand $4.10^{-3}$ mmol | 2ème ligand de formule 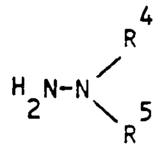 ($5.10^{-3}$ à $1.10^{-2}$ mmol) | | |
|---|---|---|---|---|
| | | NOM | $R^4$ | $R^5$ |
| 1 | triphényl phosphine | S-méthyl dithio carbazate | H | 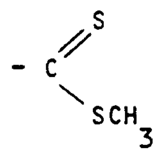 |
| 2 | " | S-méthyl N-méthyl dithiocarbazate | $CH_3$ | 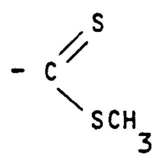 |
| 3 | " | hydrazine sous forme de dichlorhydrate $NH_2-NH_2,2HCl$ | H | H |
| 4 | " | semi-carbazide sous forme de chlorhydrate 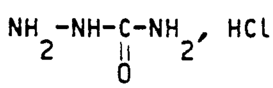 | H | 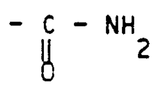 |
| 5 | " | thiosemicarbazide | H | 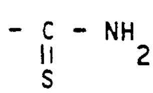 |
| 6 | " | 4-méthyl-3-thiosemicarbazide | H | 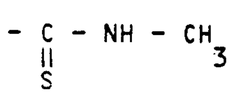 |

TABLEAU 1 (suite)

| EX | 1er ligand $4.10^{-3}$ mmol | 2ème ligand de formule 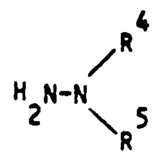 ($5.10^{-3}$ à $1.10^{-2}$ mmol) | | |
|---|---|---|---|---|
| | | NOM | $R^4$ | $R^5$ |
| 7 | triphényl phosphine | carbohydrazide | H | $-C(=O)-NH-NH_2$ |
| 8 | " | thiocarbohydrazide | H | $-C(=S)-NH-NH_2$ |
| 9 | " | acéthydrazide | H | $-C(=O)-CH_3$ |
| 10 | " | méthyl hydrazine carboxylate | H | $-C(=O)-OCH_3$ |
| 11 | " | furoyl-2-hydrazide | H | 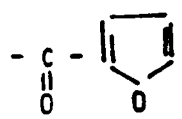 |
| 12 | " | Salicyloyl hydrazide | H | 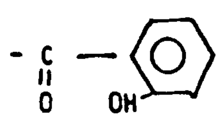 |
| 13 | " | aminoguanidine | H | $-C(=NH)-NH_2$ |

TABLEAU 2

| EX | 1er ligand | 2ème ligand 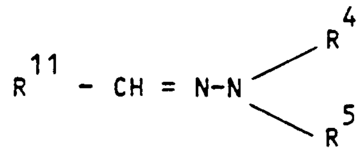 | | | |
|---|---|---|---|---|---|
| | | NOM | $R^4$ | $R^5$ | $R^{11}$ |
| 14 | triphényl phosphine | S-méthyl $\beta$-N(2-hydroxyphényl)méthylène dithiocarbazate | H | - C - $SCH_3$<br>S | 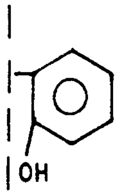 |
| 15 | " | $\alpha$ -N-méthyl S-méthyl $\beta$-N(2-hydroxyphényl) méthylène dithiocarbazate | $CH_3$ | " | " |
| 16 | " | $\alpha$,N-méthyl-S-méthyl $\beta$-N pyridylméthylène dithiocarbazate | " | " | 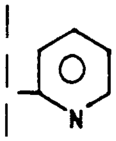 |
| 17 | diéthyl phényl phosphine | S-méthyl $\beta$,N(2-hydrophényl) méthylène dithiocarbazate | H | " | 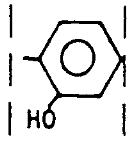 |

TABLEAU 2 (Suite)

| EX | 1er ligand | 2ème ligand 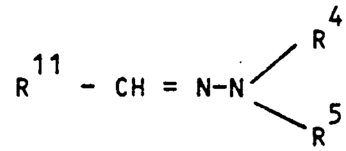 | | | |
|---|---|---|---|---|---|
| | | NOM | $R^4$ | $R^5$ | $R^{11}$ |
| 18 | triphényl phosphine | S-méthyl- β,N(2,5-dihydroxy-phényl) méthylène dithiocarbazate | H | - C - $SCH_3$<br>S | 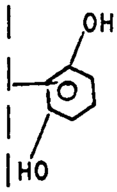 |
| 19 | diéthyl phényl phosphine | " | " | " | " |
| 20 | triéthyl phosphine | " | " | " | " |
| 21 | triméthyl phosphine | " | " | " | " |
| 22 | triphényl phosphine trisulfonée | α-N-méthyl S-méthyl β-N(2-hydroxyphényl) méthylène dithiocarbazate | $CH_3$ | " | 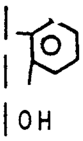 |

TABLEAU 3

| | Ex.49 | Ex.50 | Ex.51 | Ex.52 | Ex.53 | Ex.54 | Ex.55 |
|---|---|---|---|---|---|---|---|
| Organes entiers | Complexe de l'exemple 2 | Complexe de l'exemple 14 | Complexe de l'exemple 17 | Complexe de l'exemple 21 | Complexe de l'exemple 28 | Complexe de l'exemple 37 | Complexe de l'exemple 47 |
| FOIE | 19,71 | 32,41 | 35,64 | 20,23 | 12,42 | 13,63 | 7,30 |
| REINS | 7,02 | 0,80 | 2,09 | 4,54 | 14,70 | 19,61 | 0,87 |
| POUMONS | 5,54 | 2,89 | 2,07 | 2,86 | 1,11 | 0,84 | 12,64 |
| CERVEAU | 0,22 | 0,06 | 0,15 | 0,56 | 0,08 | 0,06 | 0,19 |
| COEUR | 0,82 | 0,48 | 0,90 | 0,67 | 0,35 | 0,24 | 0,23 |
| SANG | 18,03 | 32,22 | 17,58 | 15,06 | 7,56 | 10,38 | 0,84 |

## Revendications

**1.** Procédé de préparation d'un produit radiopharmaceutique comprenant un complexe nitruro d'un métal de transition choisi parmi $^{99m}Tc$, $^{186}Re$ et $^{188}Re$, comportant une partie M≡N avec M représentant le

métal de transition choisi parmi $^{99m}Tc$, $^{186}Re$ et $^{188}Re$, caractérisé en ce que l'on fait réagir un composé oxygéné $MO_4^-$ du métal de transition M avec un premier ligand choisi dans le groupe des phosphines et polyphosphines aliphatiques et aromatiques, substituées ou non substituées, et un second ligand azoté constitué, soit par un azoture d'ammonium ou de métal pharmaceutiquement acceptable, soit par un composé azoté comportant un motif $>N\text{-}N<$ dans lequel les N sont reliés à des atomes d'hydrogène et/ou à des groupements organiques monovalents par l'intermédiaire d'un atome de carbone ou dans lequel l'un des N est relié à l'atome de carbone d'un groupe organique bivalent par l'intermédiaire d'une double liaison et l'autre N est relié à des atomes d'hydrogène et/ou à des groupements organiques monovalents par l'intermédiaire d'un atome de carbone.

**2.** Procédé selon la revendication 1, caractérisé en ce que l'on effectue la réaction en solution aqueuse.

**3.** Procédé de préparation d'un produit radiopharmaceutique comprenant un complexe nitruro d'un métal de transition choisi parmi $^{99m}Tc$, $^{186}Re$ et $^{188}Re$, comportant une partie M≡N dans laquelle M représente le métal de transition choisi parmi $^{99m}Tc$, $^{186}Re$ et $^{188}Re$, caractérisé en ce que l'on fait réagir un composé oxygéné $MO^-_4$ du métal de transition M avec :

1) - un premier ligand choisi dans le groupe des phosphines et polyphosphines aliphatiques et aromatiques, substituées ou non substituées,

2) - un second ligand azoté constitué, soit par un azoture d'amonium ou de métal pharmaceutiquement acceptable, soit par un composé azoté comportant un motif $>N\text{-}N<$ dans lesquels les N sont reliés à des atomes d'hydrogène et/ou à des groupements organiques monovalents par l'intermédiaire d'un atome de carbone, ou dans lequel l'un des N est relié à l'atome de carbone d'un groupe organique bivalent par l'intermédiaire d'une double liaison et l'autre N est relié à des atomes d'hydrogène et/ou à des groupements organiques monovalents par l'intermédiaire d'un atome de carbone, et

3) - un troisième ligand organique à groupement nucléophile, un anticorps monoclonal ou un fragment d'anticorps.

**4.** Procédé selon la revendication 3, caractérisé en ce que, dans une première étape, on fait réagir le composé oxygéné du métal de transition avec le premier et le second ligands, et, dans une deuxième étape, on fait réagir le produit obtenu à la suite de la première étape avec le troisième ligand, l'anticorps monoclonal ou le fragment d'anticorps.

**5.** Procédé selon la revendication 3, caractérisé en ce qu'au moins l'une des réactions est effectuée en solution aqueuse.

**6.** Procédé selon la revendication 3, caractérisé en ce que l'on fait réagir simultanément les trois ligands en solution aqueuse.

**7.** Procédé selon l'une quelconque des revendications 3 à 6, caractérisé en ce que le troisième ligand est choisi parmi les amines, les thiols, les thioéthers, les oximes, les phosphines, et les ligands polyfonctionnels du type polyaminopolythiol.

**8.** Procédé selon l'une quelconque des revendications 3 à 6, caractérisé en ce que le troisième ligand est choisi parmi le 1,1'-(1,2-éthane diyl-diimino)-bis-(2-méthyl-2-propane thiol) le tétraazaundécane et le 1,2,bis(diméthylphosphine)éthane hydrochlorure et le chrorhydrate de la 8-mercapto quinoléine.

**9.** Procédé selon l'une quelconque des revendications 3 à 6, caractérisé en ce que l'anticorps monoclonal est un anticorps anti-ACE.

**10.** Procédé selon l'une quelconque des revendications 1 à 9, caractérisé en ce que le composé oxygéné du métal de transition est un pertechnétate -99m de métal alcalin ou d'ammonium.

**11.** Procédé selon l'une quelconque des revendications 1 à 9, caractérisé en ce que le composé oxygéné du métal de transition est du perrhénate -186 ou -188 de métal alcalin ou d'ammonium.

**12.** Procédé selon l'une quelconque des revendications 1 à 11, caractérisé en ce que le premier ligand est une phosphine répondant à la formule :

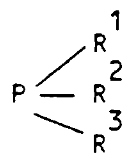

dans laquelle $R^1$, $R^2$ et $R^3$ qui peuvent être identiques ou différents, représentent un atome d'hydrogène, un radical alkyle, un radical aryle, un radical alcoxy ou un radical alkyle ou aryle substitué par un groupement choisi parmi les radicaux amino, amido, cyano et sulfonate.

**13.** Procédé selon l'une quelconque des revendications 1 à 11, caractérisé en ce que le premier ligand est une polyphosphine répondant aux formules :

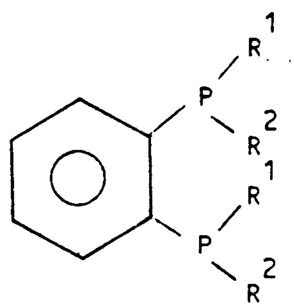

ou

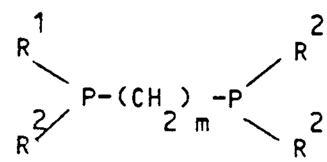

dans lesquelles $R^1$ et $R^2$ qui peuvent être identiques ou différents, représentent un atome d'hydrogène, un radical alkyle, un radical aryle, un radical alcoxy ou un radical alkyle ou aryle substitué par un groupement choisi parmi les radicaux amino et amido, et m est un nombre entier allant de 1 à 4.

**14.** Procédé selon la revendication 12, caractérisé en ce que le premier ligand est une phosphine choisie parmi la triphénylphosphine, la diéthylphénylphosphine, la triéthylphosphine, la triméthylphosphine et la tris(cyanoéthyl-2) phosphine.

**15.** Procédé selon l'une quelconque des revendications 1 à 14, caractérisé en ce que le second ligand azoté est un azoture de métal alcalin.

**16.** Procédé selon l'une quelconque des revendications 1 à 14, caractérisé en ce que le second ligand est un composé azoté répondant à la formule :

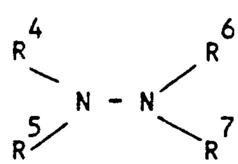

dans laquelle $R^4$, $R^5$, $R^6$ et $R^7$ qui peuvent être identiques ou différents, représentent un atome d'hydrogène ; un radical alkyle ; un radical aryle ; un radical alcoxy ; un radical alkyle substitué par au moins un groupement choisi parmi les radicaux hydroxy, carboxy, amino, amido et mercapto ; un radical aryle substitué par au moins un groupement choisi parmi les atomes d'halogène et les radicaux

alcoxy, hydroxy, amino, mercapto et amino substitué par au moins un radical alkyle ; un radical répondant aux formules :

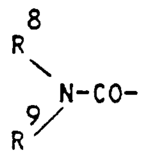 , 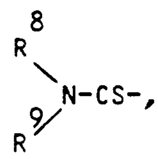 , ou 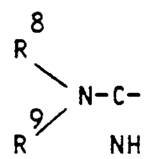

dans lesquelles $R^8$ et $R^9$ qui peuvent être identiques ou différents, représentent un atome d'hydrogène, un radical alkyle, ou un radical amino ; un radical de formule :

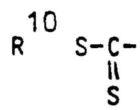

dans laquelle $R^{10}$ représente un atome d'hydrogène, un radical alkyle ou un radical aryle ; un radical de formule $R^{11}$ -CO- avec $R^{11}$ représentant un radical alkyle, un radical alcoxy, un radical aryle non substitué ou substitué par au moins un groupement choisi parmi les atomes d'halogène et les radicaux hydroxy, ou un radical dérivé d'un hétérocycle non substitué ou substitué par au moins un groupement choisi parmi les atomes d'halogène et les radicaux hydroxy ; ou dans laquelle $R^4$ et $R^5$ peuvent former ensemble un radical bivalent de formule :

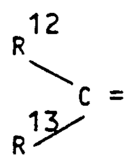

dans laquelle $R^{12}$ représente $-CH_2-NH_2$, un radical aryle non substitué ou substitué par au moins un groupement choisi parmi les atomes d'halogène et les radicaux hydroxy, alcoxy, amino, mercapto et amino substitué par au moins un radical alkyle, ou un radical dérivé d'un hétérocycle non substitué ou substitué par un ou plusieurs groupements choisis parmi les atomes d'halogène et les radicaux hydroxy, alcoxy, amino, mercapto et amino substitué par au moins un radical alkyle et, $R^{13}$ représente un atome d'hydrogène, un radical alkyle ou un radical alkyle substitué par au moins un groupement choisi parmi les radicaux hydroxy, carboxy, amino, amido et mercapto ; et $R^6$ et $R^7$ ont la signification donnée ci-dessus.

**17.** Procédé selon l'une quelconque des revendications 1 à 14, caractérisé en ce que le second ligand est l'acide dithiocarbazique ou un dérivé de celui-ci répondant à la formule :

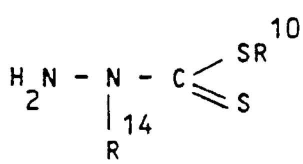

dans laquelle $R^{10}$ représente un atome d'hydrogène, un radical alkyle ou un radical aryle, et $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alcoxy, hydroxy, amino, mercapto et amino substitué par au moins un radical alkyle.

**18.** Procédé selon l'une quelconque des revendications 1 à 14, caractérisé en ce que le second ligand est un produit de condensation de l'acide dithiocarbazique répondant à la formule :

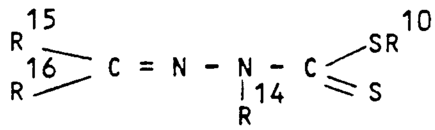

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alcoxy, hydroxy, amino, mercapto et amino substitué par au moins un radical alkyle ; et $R^{15}$ et $R^{16}$ qui peuvent être identiques ou différents représentent un atome d'hydrogène, un radical alkyle ou un radical alkyle substitué par au moins un groupement choisi parmi les radicaux hydroxy, carboxy, amino, amido et mercapto.

**19.** Procédé selon l'une quelconque des revendications 1 à 14, caractérisé en ce que le second ligand est un produit de condensation de l'acide dithiocarbazique répondant à la formule :

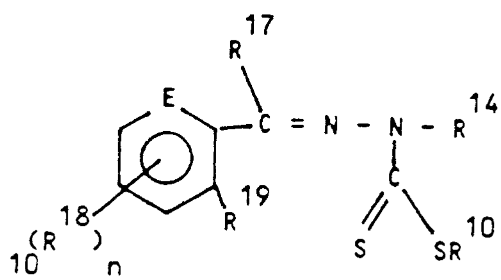

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alcoxy, hydroxy, amino, mercapto et amino substitué par au moins un radical alkyle ; $R^{17}$ représente un atome d'hydrogène, un radical alkyle, un radical alkyle substitué par au moins un groupement choisi parmi les radicaux hydroxy, carboxy, amino, amido et mercapto, $R^{18}$ représente un atome d'hydrogène, un atome d'halogène, un radical alcoxy, un radical amino, ou un radical amino substitué par au moins un groupement alkyle, $R^{19}$ représente un atome d'hydrogène, un radical hydroxy ou un radical mercapto, E représente un atome de carbone ou un atome d'azote, et n est un nombre entier allant de 1 à 4, ou dans laquelle n est égal à 2 et les deux $R^{18}$ sont voisins et forment ensemble un cycle aromatique.

**20.** Procédé selon l'une quelconque des revendications 1 à 14, caractérisé en ce que le second ligand est un produit de condensation de l'acide dithiocarbazique répondant à la formule :

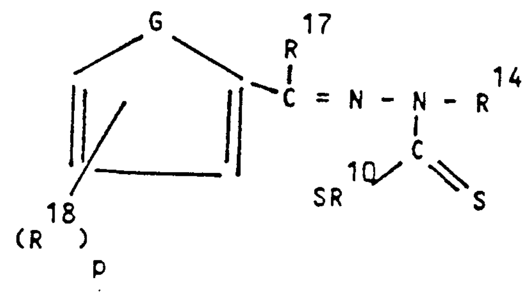

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alcoxy, hydroxy, amino mercapto et amino substitué par au moins un radical alkyle ; $R^{17}$ représente un atome d'hydrogène, un radical alkyle, un radical alkyle substitué par au moins un groupement choisi parmi les radicaux hydroxy, carboxy, amino, amido et mercapto, $R^{18}$ représente un atome d'hydrogène, un atome d'halogène, un radical alcoxy, un radical amino, ou un radical amino substitué par au moins un groupement alkyle, G est S ou O, et p est 1, 2 ou 3.

**21.** Procédé selon l'une quelconque des revendications 1 à 14, caractérisé en ce que le second ligand est l'hydrazine ou un dérivé d'hydrazine répondant à la formule :

$R^{20}$-NH-NH-$R^{21}$

dans laquelle $R^{20}$ est un atome d'hydrogène ou un radical alkyle et $R^{21}$ est un atome d'hydrogène ou un radical choisi parmi les radicaux de formule :

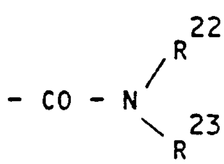

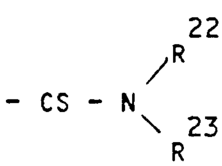

-CO $R^{24}$
-COO $R^{24}$
-CO-$R^{25}$

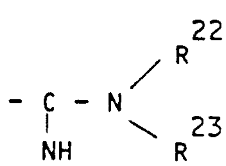

dans lesquelles $R^{22}$ et $R^{23}$ qui peuvent être identiques ou différents, représentent un atome d'hydrogène, un radical alkyle ou un radical amino, $R^{24}$ représente un radical alkyle et $R^{25}$ représente un radical aryle non substitué ou substitué par au moins un groupement choisi parmi les atomes d'halogène et les radicaux hydroxy ou un radical dérivé d'un hétérocycle non substitué ou substitué par au moins un groupement choisi parmi les atomes d'halogène et les radicaux hydroxy.

**22.** Procédé selon l'une quelconque des revendications 1 à 14, caractérisé en ce que le second ligand est choisi parmi le S-méthyl-bêta-N(2-hydroxyphényl) méthylène dithiocarbazate, le S-méthyldithiocarbazate, le S-méthyl N-méthyldithiocarbazate, l'alpha-N-méthyl-S-méthyl-bêta-N-pyridylméthylène dithio-carbazate, le S-méthyl-bêta-N-(2,5-dihydroxyphényl) méthylène dithio-carbazate, le alpha-N-méthyl-S-méthyl-bêta-N(2-hydroxyphényl) méthylène dithiocarbazate, l'hydrazine, le semicarbazide, le thiosemicarbazide, le 1-méthyl-3-thiosemicarbazide, le 4-méthyl-3-thiosemicarbazide, l'amino-acétonesemicarbazone, le carbohydrazide, le thiocarbohydrazide, l'acéthydrazide, le méthylhydrazinecarboxylate, le furoyl-2 hydrazide, le salicyloyl hydrazide, l'aminoguanidine et l'azoture de sodium.

**23.** Trousse pour la préparation d'un complexe nitruro de technétium par mise en oeuvre du procédé selon l'une quelconque des revendications 1 à 22, caractérisée en ce qu'elle comprend :
- un premier flacon contenant le premier ligand, et
- un second flacon contenant le second ligand azoté.

**24.** Trousse selon la revendication 23, caractérisée en ce qu'elle comprend un troisième flacon contenant un troisième ligand organique à groupement nucléophile, un anticorps monoclonal ou un fragment d'anticorps.

**Claims**

**1.** Process for the preparation of a radiopharmaceutical product incorporating a nitride complex of a transition metal chosen from among $^{99m}Tc$, $^{186}Re$ and $^{188}Re$, having a portion M≡N with M representing the transition metal chosen from among $^{99m}Tc$, $^{186}Re$ and $^{188}Re$, characterized in that an oxidized compound $MO_4^-$ of the transition metal M is reacted with a first ligand chosen from the group of substituted or unsubstituted, aliphatic and aromatic phosphines and polyphosphines, and a second nitrogenous ligand constituted either by an ammonium nitride or pharmaceutically acceptable metal, or a nitrogenous compound having a >N-N< unit, in which the N's are linked with the hydrogen atoms and/or monovalent organic groups via a carbon atom, or in which one of the ends is linked with the carbon atom of a divalent organic group via a double bond and the other N is linked with the hydrogen atoms and/or monovalent organic groups via a carbon atom.

**2.** Process according to claim 1, characterized in that the reaction is performed in an aqueous solution.

**3.** Process for the preparation of a radiopharmaceutical product incorporating a nitride complex of a transition metal chosen from among $^{99m}Tc$, $^{186}Re$ and $^{188}Re$, having a portion M≡N, in which M represents the transition metal chosen from among $^{99m}Tc$, $^{186}Re$ and $^{188}Re$, characterized in that an oxidized compound $MO_4^-$ of the transition metal M is reacted with:

1) a first ligand chosen from the group of substituted or unsubstituted, aliphatic and aromatic phosphines and polyphosphines,

2) a second nitrogenous ligand constituted either by an ammonium nitride or a pharmaceutically acceptable metal, or a nitrogenous compound having a >N-N< unit, in which the N's are linked to hydrogen atoms and/or monovalent organic groups via a carbon atom, in which one of the ends is linked to the carbon atom by a divalent organic group via a double bond and the other N is linked with the hydrogen atoms and/or monovalent organic groups via a carbon atom and

3) a third organic ligand with a nucleophilic group, a monoclonal antibody or an antibody fragment.

**4.** Process according to claim 3, characterized in that, in a first stage, the oxidized compound of the transition metal is reacted with the first and second ligands and that, in a second stage, the product obtained from the first stage is reacted with the third ligand, the monoclonal antibody or the antibody fragment.

**5.** Process according to claim 3, characterized in that at least one of the reactions is performed in an aqueous solution.

**6.** Process according to claim 3, characterized in that the three ligands in aqueous solution are reacted simultaneously.

**7.** Process according to any one of the claims 3 to 6, characterized in that the third ligand is chosen from

among amines, thiols, thioethers, oximes, phosphines and polyfunctional ligands of the polyaminopolythiol type.

**8.** Process according to any one of the claims 3 to 6, characterized in that the third ligand is chosen from among 1,1'-(1,2-ethane diyl-diimino)-bis-(2-methyl-2-propane thiol), tetraazaundecane and 1,2-bis-(dimethyl phosphine)ethane hydrochloride and 8-mercapto quinoline hydrochloride.

**9.** Process according to any one of the claims 3 to 6, characterized in that the monoclonal antibody is an anti-ACE antibody.

**10.** Process according to any one of the claims 1 to 9, characterized in that the oxidized compound of the transition metal is a -99m pertechnetate of alkali metal or ammonium.

**11.** Process according to any one of the claims 1 to 9, characterized in that the oxidized compound of the transition metal is -186 or -188 perrhenate of alkali metal or ammonium.

**12.** Process according to any one of the claims 1 to 11, characterized in that the first ligand is a phosphine complying with the formula:

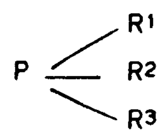

in which $R^1$, $R^2$ and $R^3$, which can be the same or different, represent a hydrogen atom, an alkyl radical, an aryl radical, an alkoxy radical or an alkyl or aryl radical substituted by a group chosen from among amino, amido, cyano and sulphonate radicals.

**13.** Process according to any one of the claims 1 to 11, characterized in that the first ligand is a polyphosphine complying with the formulas;

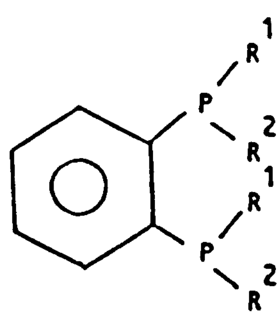

or

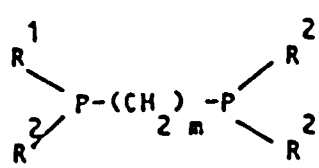

in which $R^1$, and $R^2$, which can be the same or different, represent a hydrogen atom, an alkyl radical, an aryl radical, an alkoxy radical or an alkyl or aryl radical substituted by a group chosen from among the amino and amido radicals and m is an integer between 1 and 4.

**14.** Process according to claim 12, characterized in that the first ligand is a phosphine chosen from among triphenyl phosphine, diethyl phenyl phosphine, triethyl phosphine, trimethyl phosphine and tris(2-cyanoethyl)-phosphine.

**15.** Process according to any one of the claims 1 to 14, characterized in that the second nitrogenous ligand is an alkali metal nitride.

**16.** Process according to any one of the claims 1 to 14, characterized in that the second ligand is a nitrogenous compound complying with the formula;

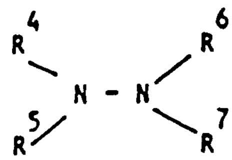

in which $R^4$, $R^5$, $R^6$ and $R^7$, which can be the same or different, represent a hydrogen atom, an alkyl radical, an aryl radical, an alkoxy radical, an alkyl radical substituted by at least one group chosen from among the hydroxy, carboxy, amino, amido and mercapto radicals, an aryl radical substituted by at least one group chosen from among the halogen atoms and alkoxy, hydroxy, amino and mercapto radical or amine radical substituted by at least one alkyl radical, a radical in accordance with the formulas:

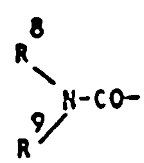 , 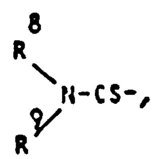 or 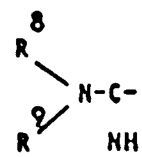

in which $R^8$ and $R^9$, which can be the same or different, represent a hydrogen atom, an alkyl radical or an amino radical, a radical of formula:

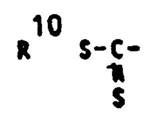

in which $R^{10}$ represents a hydrogen atom, an alkyl radical or an aryl radical, a radical of formula $R^{11}$-CO- with $R^{11}$ representing an alkyl radical, an alkoxy radical, an aryl radical not substituted or substituted by at least one group chosen from among the halogen atoms and the hydroxy radicals, or a radical derived from a heterocycle which is not substituted or substituted by at least one group chosen from among the halogen atoms and the hydroxy radicals, or in which $R^4$ and $R^5$ can together form a divalent radical of formula:

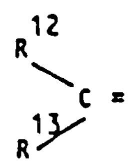

in which $R^{12}$ represents $-CH_2-NH_2$, an aryl radical not substituted or substituted by at least one group chosen from among the halogen atoms and the alkoxy, hydroxy, amino and mercapto radical or amino radical substituted by at least one alkyl radical, or a radical derived from a heterocycle, which is not substituted or substituted by one or more groups chosen from among halogen atoms and hydroxy, alkoxy, amino and mercapto radicals and amino radicals substituted by at least one alkyl radical and $R^{13}$ represents a hydrogen atom, an alkyl radical or an alkyl radical substituted by at least one group chosen from among hydroxy, carboxy, amino, amido and mercapto radicals and $R^6$ and $R^7$ have the

meanings given hereinbefore.

**17.** Process according to any one of the claims 1 to 14, characterized in that the second ligand is dithiocarbazic acid or a derivative thereof complying with the formula:

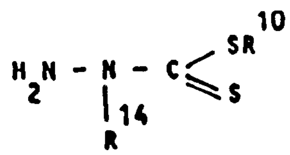

in which $R^{10}$ represents a hydrogen atom, an alkyl radical or an aryl radical and $R^{14}$ represents a hydrogen atom, an alkyl radical, an aryl radical, an alkoxy radical, an alkyl radical substituted by at least one group chosen from among hydroxy, carboxy, amino, amido and mercapto radicals, or an aryl radical substituted by at least one group chosen from among the halogen atoms and the alkoxy, hydroxy, amino and mercapto radicals and the amino radical substituted by at least one alkyl radical.

**18.** Process according to any one of the claims 1 to 14, characterized in that the second ligand is a condensation product of dithiocarbazic acid in accordance with the formula:

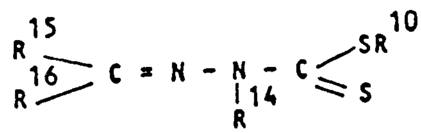

in which $R^{10}$ represents a hydrogen atom, an alkyl radical or an aryl radical, $R^{14}$ represents a hydrogen atom, an alkyl radical, an aryl radical, an alkoxy radical, an alkyl radical substituted by at least one group chosen from among the hydroxy, carboxy, amino, amido and mercapto radicals, or an aryl radical substituted by at least one group chosen from among the halogen atoms and the alkoxy, hydroxy, amino and mercapto radicals and the amino radicals substituted by at least one alkyl radical and $R^{15}$ and $R^{16}$, which can be the same or different, represent a hydrogen atom, an alkyl radical or an alkyl radical substituted by at least one group chosen from among the hydroxy, carboxy, amino, amido and mercapto radicals.

**19.** Process according to any one of the claims 1 to 14, characterized in that the second ligand is a condensation product of dithiocarbazic acid in accordance with the formula:

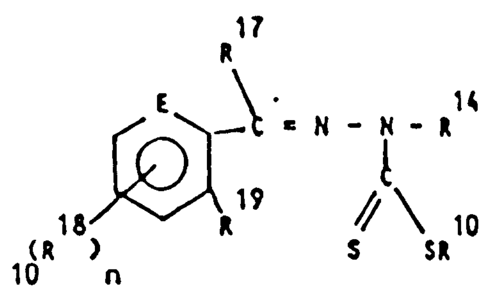

in which $R^{10}$ represents a hydrogen atom, an alkyl radical or an aryl radical, $R^{14}$ represents a hydrogen atom, an alkyl radical, an aryl radical, an alkoxy radical, an alkyl radical substituted by at least one group chosen from the hydroxy, carboxy, amino, amido and mercapto radicals or an aryl radical substituted by at least one group chosen from among halogen atoms and alkoxy, hydroxy, amino and mercapto radicals and the amino radical substituted by at least one alkyl radical, $R^{17}$ represents a hydrogen atom, an alkyl radical, an alkyl radical substituted by at least one group chosen from among the hydroxy, carboxy, amino, amido and mercapto radicals, $R^{18}$ represents a hydrogen atom, a halogen

atom, an alkoxy radical, an amino radical or an amino radical substituted by at least one alkyl group, $R^{19}$ represents a hydrogen atom, a hydroxy radical or a mercapto radical, E represents a carbon atom or a nitrogen atom and n is an integer between 1 and 4, or in which n is equal to 2 and the two $R^{18}$ are close to one another and together form an aromatic cycle.

**20.** Process according to any one of the claims 1 to 14, characterized in that the second ligand is a condensation product of dithiocarbazic acid in accordance with the formula:

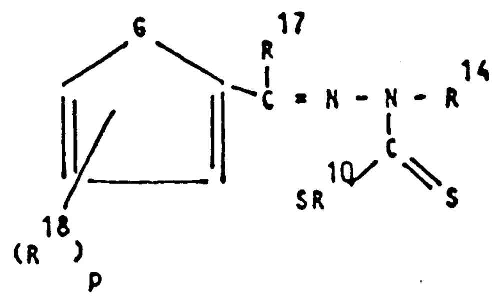

in which $R^{10}$ represents a hydrogen atom, an alkyl radical or an aryl radical, $R^{14}$ represents a hydrogen atom, an alkyl radical, an aryl radical, an alkoxy radical, an alkyl radical substituted by at least one group chosen from among the hydroxy, carboxy, amino, amido and mercapto radicals, or an aryl radical substituted by at least one group chosen from among the halogen atoms and the alkoxy, hydroxy, amino and mercapto radicals and the amino radicals substituted by at least one alkyl radical, $R^{17}$ represents a hydrogen atom, an alkyl radical, an alkyl radical substituted by at least one group chosen from among the hydroxy, carboxy, amino, amido and mercapto radicals, $R^{18}$ represents a hydrogen atom, a halogen atom, an alkoxy radical, an amino radical, or an amino radical substituted by at least one alkyl group, G is S or O and p is 1, 2 or 3.

**21.** Process according to any one of the claims 1 to 14, characterized in that the second ligand is hydrazine or a hydrazine derivative complying with the formula:

$R^{20}$-NH-NH-$R^{21}$

in which $R^{20}$ is a hydrogen atom or an alkyl radical and $R^{21}$ is a hydrogen atom or a radical chosen from among the radicals of formula:

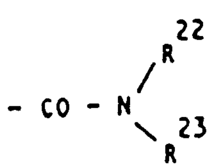

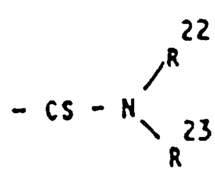

-CO $R^{24}$
-COO $R^{24}$
-CO-$R^{25}$

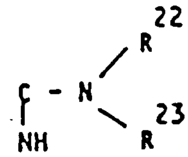

in which $R^{22}$ and $R^{23}$, which can be the same or different, represent a hydrogen atom, an alkyl radical or an amino radical, $R^{24}$ represents an alkyl radical and $R^{25}$ represents an aryl radical, which is not substituted or substituted by at least one group chosen from among the halogen atoms and the hydroxy radicals or a radical derived from a heterocycle, which is not substituted or substituted by at least one group chosen from among the halogen atoms and the hydroxy radicals.

**22.** Process according to any one of the claims 1 to 14, characterized in that the second ligand is chosen from among S-methyl-beta-N(2-hydroxyphenyl) methylene dithiocarbazate, S-methyldithiocarbazate, S-methyl N-methyldithiocarbazate, alpha-N-methyl-S-methyl-beta-N-pyridylmethylene dithiocarbazate, S-methyl-beta-N-(2,5-dihydroxyphenyl) methylene dithiocarbazate, alpha-N-methyl-S-methyl-beta-N(2-hydroxyphenyl) methylene dithiocarbazate, hydrazine, semicarbazide, thiosemicarbazide, 1-methyl-3-thiosemicarbazide 4-methyl-3-thiosemicarbazide, aminoacetonesemicarbazone, carbohydrazide, thiocarbohydrazide, acetohydrazide, methylhydrazinecarboxylate 2-furoyl hydrazide, salicyloyl hydrazide, aminoguanidine and sodium nitride.

**23.** Kit for the preparation of a technetium nitride complex by performing the process according to any one of the claims 1 to 22, characterized in that it comprises a first bottle containing the first ligand and a second bottle containing the second nitrogenous ligand.

**24.** Kit according to claim 23, characterized in that it comprises a third bottle containing a third organic ligand with a nucleophilic group, a monoclonal antibody or an antibody fragment.

## Patentansprüche

**1.** Verfahren zur Herstellung eines radiopharmazeutischen Produkts, enthaltend einen Nitridokomplex eines aus $^{99m}$Tc, $^{186}$Re und $^{188}$Re ausgewählten Übergangsmetalls, welcher einen Teil M≡N enthält, wobei M das aus $^{99m}$Tc, $^{186}$Re und $^{188}$Re ausgewählte Übergangsmetall darstellt, dadurch gekennzeichnet, daß man eine Sauerstoffverbindung $MO_4$ des Übergangsmetalls M mit einem ersten aus der Gruppe der substituierten oder nichtsubstituierten, aliphatischen und aromatischen Phosphine und Polyphosphine ausgewählten Liganden und einem zweiten Stickstoffliganden reagieren läßt, welcher entweder aus einem Ammoniumazid oder einem Azid eines pharmazeutisch annehmparen Metalls oder aus einer Stickstoffverbindung besteht, welche eine Struktureinheit $>$N-N$<$ enthält, in welcher die N an Wasserstoffatome und/oder durch ein Zwischenglied aus einem Kohlenstoffatom an einwertige organische Gruppierungen gebunden sind, oder in welcher eines der N durch ein Zwischenglied aus einer Doppelbindung an das Kohlenstoffatom einer zweiwertigen organischen Gruppierung gebunden ist und das andere N an Wasserstoffatome und/oder durch ein Zwischenglied aus einem Kohlenstoffatom an einwertige organische Gruppierungen gebunden ist.

**2.** Verfahren gemäß Anspruch 1, dadurch gekennzeichnet, daß man die Reaktion in wäßriger Lösung durchführt.

**3.** Verfahren zur Herstellung eines radiopharmazeutischen Produkts, enthaltend einen Nitridokomplex eines unter $^{99m}$Tc, $^{186}$Re und $^{188}$Re ausgewählten Übergangsmetalls, welcher einen Teil M≡N enthält, in welchem das M das unter $^{99m}$Tc, $^{186}$Re und $^{188}$Re ausgewählte Übergangsmetall darstellt, dadurch gekennzeichnet, daß man eine Sauerstoffverbindung $MO_4$ des Übergangsmetalls M mit:

1) - einem ersten aus der Gruppe der aliphatischen und aromatischen, substituierten oder nichtsubstituierten Phosphine und Polyphosphine ausgewählten Liganden,

2) - einem zweiten Stickstoffliganden, welcher entweder aus einem Ammoniumazid oder einem Azid eines pharmazeutisch annehmbaren Metalls oder aus einer Stickstoffverbindung besteht, welche eine Struktureinheit $>$N-N$<$ enthält, in welcher die N an Wasserstoffatome und/oder durch ein Zwischenglied aus einem Kohlenstoffatom an einwertige organische Gruppierungen gebunden sind,

oder in welcher eines der N durch ein Zwischenglied aus einer Doppelbindung an das Kohlenstoffatom einer zweiwertigen organischen Gruppierung gebunden ist und das andere N an Wasserstoffatome und/oder durch ein Zwischenglied aus einem Kohlenstoffatom an einwertige organische Gruppierungen gebunden ist, und

3) - einem dritten organischen Liganden mit einer nukleophilen Gruppe, einem monoklonalen Antikörper oder einem Antikörperbruchstück reagieren läßt.

4. Verfahren gemäß Anspruch 3, dadurch gekennzeichnet, daß man in einem ersten Schritt die Sauerstoffverbindung des Übergangsmetalls mit dem ersten und zweiten Liganden reagieren läßt und man in einem zweiten Schritt das aus dem ersten Schritt erhaltene Produkt mit dem dritten Liganden, dem monoklonalen Antikörper oder dem Antikörperbruchstück reagieren läßt.

5. Verfahren gemäß Anspruch 3, dadurch gekennzeichnet, daß wenigstens eine der Reaktionen in wäßriger Lösung durchgeführt wird.

6. Verfahren gemäß Anspruch 3, dadurch gekennzeichnet, daß man die drei Liganden gleichzeitig in wäßriger Lösung reagieren läßt.

7. Verfahren gemäß einem der Ansprüche 3 bis 6, dadurch gekennzeichnet, daß der dritte Ligand aus den Aminen, den Thiolen, den Thioethern, den Oximen, den Phosphinen und den polyfunktionellen Liganden vom Polyaminopolythiol-Typ ausgewählt ist.

8. Verfahren gemäß einem der Ansprüche 3 bis 6, dadurch gekennzeichnet, daß der dritte Ligand aus 1,1'-(1,2-Ethandiyl-diimino)bis-(2-methyl-2-propanthiol), Tetraazaundecan und 1,2-Bis(dimethylphosphin)ethan-hydrochlorid und 8-Mercaptochinolin-hydrochlorid ausgewählt ist.

9. Verfahren gemäß einem der Ansprüche 3 bis 6, dadurch gekennzeichnet, daß der monoklonale Antikörper ein Anti-ACE-Antikörper ist.

10. Verfahren gemäß einem der Ansprüche 1 bis 9, dadurch gekennzeichnet, daß die Sauerstoffverbindung des Übergangsmetalls ein Alkalimetall- oder Ammonium-pertechnetat -99m ist.

11. Verfahren gemäß einem der Ansprüche 1 bis 9, dadurch gekennzeichnet, daß die Sauerstoffverbindung des Übergangsmetalls ein Alkalimetall- oder Ammonium-perrhenat -186 oder -188 ist.

12. Verfahren gemäß einem der Ansprüche 1 bis 11, dadurch gekennzeichnet, daß der erste Ligand ein Phosphin ist, welches der Formel

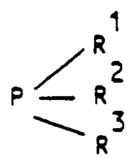

entspricht, worin $R^1$, $R^2$ und $R^3$, welche identisch oder verschieden sein können, ein Wasserstoffatom, einen Alkylrest, einen Arylrest, einen Alkoxyrest oder einen durch eine aus den Amino-, Amido-, Cyan- oder Sulfonatresten ausgewählte Gruppierung substituierten Alkyl- oder Arylrest bedeuten.

13. Verfahren gemäß einem der Ansprüche 1 bis 11, dadurch gekennzeichnet, daß der erste Ligand ein Polyphosphin ist, welches der Formel

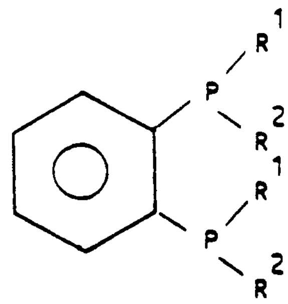

oder

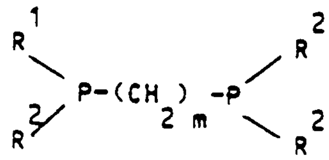

entspricht, worin $R^1$ und $R^2$, welche identisch oder verschieden sein können, ein Wasserstoffatom, einen Alkylrest, einen Arylrest, einen Alkoxyrest oder einen durch eine aus den Amino- und Amidoresten ausgewählte Gruppierung substituierten Alkyl- oder Arylrest bedeuten, und m eine ganze Zahl von 1 bis 4 ist.

**14.** Verfahren gemäß Anspruch 12, dadurch gekennzeichnet, daß der erste Ligand ein aus Triphenylphosphin, Diethylphenylphosphin, Triethylphosphin, Trimethylphosphin und Tris(2-cyanethyl)phosphin ausgewähltes Phosphin ist.

**15.** Verfahren gemäß einem der Ansprüche 1 bis 14, dadurch gekennzeichnet, daß der zweite Stickstoffligand ein Alkalimetallazid ist.

**16.** Verfahren gemäß einem der Ansprüche 1 bis 14, dadurch gekennzeichnet, daß der zweite Ligand eine Stickstoffverbindung ist, welche der Formel

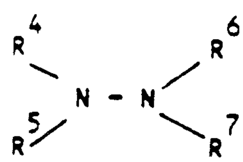

entspricht, in welcher $R^4$, $R^5$, $R^6$ und $R^7$, welche identisch oder verschieden sein können, ein Wasserstoffatom, einen Alkylrest, einen Arylrest, einen Alkoxyrest, einen durch wenigstens eine aus den Hydroxy-, Carboxy, Amino-, Amido- und Mercaptoresten ausgewählte Gruppierung substituierten Alkylrest, einen durch wenigstens eine aus den Halogenatomen und den Alkoxy-, Hydroxy-, Amino-, Mercapto- und durch wenigstens einen Alkylrest substituierten Aminoresten ausgewählte Gruppierung substituierten Arylrest; ein den Formeln

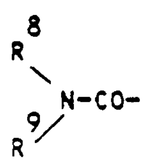 , 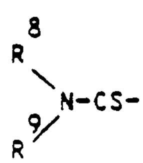 oder 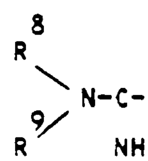

entsprechender Rest, in welchen $R^8$ und $R^9$, welche identisch oder verschieden sein können, ein Wasserstoffatom, einen Alkylrest oder einen Aminorest bedeuten; einen Rest der Formel

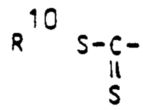

in welcher $R^{10}$ ein Wasserstoffatom, einen Alkylrest oder einen Arylrest bedeutet; einen Rest der Formel $R^{11}$-CO-, wobei $R^{11}$ einen Alkylrest, einen Alkoxyrest, einen nichtsubstituierten oder durch wenigstens eine aus den Halogenatomen und den Hydroxyresten ausgewählte Gruppierung substituierten Arylrest; oder einen von einem nichtsubstituierten oder durch eine aus den Halogenatomen und den Hydroxyresten ausgewählte Gruppierung substituierten Heterocyclus abgeleiteten Rest bedeutet; oder in welcher $R^4$ und $R^5$ zusammen einen zweiwertigen Rest der Formel

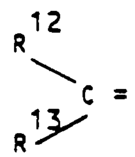

bilden können, in welchem $R^{12}$ $-CH_2-NH_2$, einen nichtsubstituierten oder durch mindestens eine aus den Halogenatomen und den Hydroxy-, Alkoxy-, Amino-, Mercapto- und durch mindestens einen Alkylrest substituierten Aminoresten ausgewählte Gruppierung substituierten Arylrest oder einen von einem nichtsubstituierten oder durch eine oder mehrere aus den Halogenatomen und den Hydroxy-, Alkoxy, Amino, Mercapto- und durch mindestens einen Alkylrest substituierten Aminoresten ausgewählte Gruppierungen substituierten Heterocyclus abgeleiteten Rest bedeutet, und $R^{13}$ ein Wasserstoffatom, einen Alkyl rest oder einen durch wenigstens eine aus den Hydroxy-, Carboxy-, Amino-, Amido- und Mercaptoresten ausgewählte Gruppierung substituierten Alkylrest bedeutet; und $R^6$ und $R^7$ die vorstehend angegebene Bedeutung besitzen.

**17.** Verfahren gemäß einem der Ansprüche 1 bis 14, dadurch gekennzeichnet, daß der zweite Ligand eine Dithiocarbazinsäure oder ein Derivat derselben ist, welche der Formel

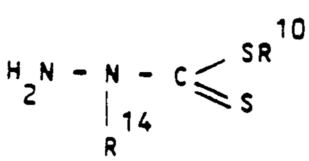

entsprechen, in welcher $R^{10}$ ein Wasserstoffatom, einen Alkylrest oder einen Arylrest bedeutet, und $R^{14}$ ein Wasserstoffatom, einen Alkylrest, einen Arylrest, einen Alkoxyrest, einen durch mindestens eine aus den Hydroxy-, Carboxy-, Amino-, Amido- und Mercaptoresten ausgewählte Gruppierung substituierten Alkylrest, oder einen durch mindestens eine aus den Halogenatomen und den Alkoxy-, Hydroxy-, Amino-, Mercapto- und wenigstens durch einen Alkylrest substituierten Aminoresten ausgewählte Gruppierung substituierten Arylrest bedeutet.

**18.** Verfahren gemäß einem der Ansprüche 1 bis 14, dadurch gekennzeichnet, daß der zweite Ligand ein Kondensationsprodukt der der Formel

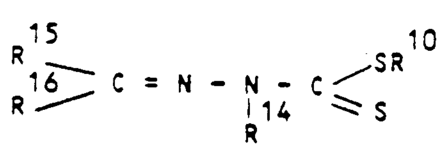

entsprechenden Dithiocarbazinsäure ist, in welcher $R^{10}$ ein Wasserstoffatom, einen Alkylrest oder einen

Arylrest bedeutet; $R^{14}$ ein Wasserstoffatom, einen Alkylrest, einen Arylrest, einen Alkoxyrest, einen durch wenigstens eine aus den Hydroxy-, Carboxy-, Amino-, Amido- und Mercaptoresten ausgewählte Gruppierung substituierten Alkylrest oder einen durch mindestens eine aus den Halogenatomen und den Alkoxy-, Hydroxy-, Amino-, Mercapto- und wenigstens durch einen Alkylrest substituierten Aminoresten ausgewählte Gruppierung substituierten Arylrest bedeutet und $R^{15}$ und $R^{16}$, welche identisch oder verschieden sein können, ein Wasserstoffatom, einen Alkylrest oder einen durch mindestens eine aus den Hydroxy-, Carboxy-, Amino-, Amido- und Mercaptoresten ausgewählte Gruppierung substituierten Alkylrest bedeuten.

**19.** Verfahren gemäß einem der Ansprüche 1 bis 14, dadurch gekennzeichnet, daß der zweite Ligand ein Kondensationsprodukt der der Formel

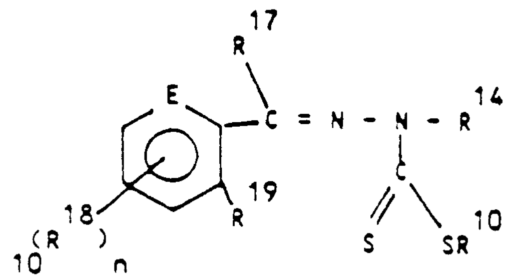

entsprechenden Dithiocarbazinsäure ist, in welcher $R^{10}$ ein Wasserstoffatom, einen Alkylrest oder einen Arylrest bedeutet; $R^{14}$ ein Wasserstoffatom, einen Alkylrest, einen Arylrest, einen Alkoxyrest, einen durch wenigstens eine aus den Hydroxy-, Carboxy-, Amino-, Amido- und Mercaptoresten ausgewählte Gruppierung substituierten Alkylrest oder einen durch mindestens eine aus den Halogenatomen und den Alkoxy-, Hydroxy-, Amino-, Mercapto- und wenigstens durch einen Alkylrest substituierten Aminoresten ausgewählte Gruppierung substituierten Arylrest bedeutet; $R^{17}$ ein Wasserstoffatom, einen Alkylrest, einen durch wenigstens eine aus den Hydroxy-, Carboxy-, Amino-, Amido- und Mercaptoresten ausgewählte Gruppierung substituierten Alkylrest bedeutet, $R^{18}$ ein Wasserstoffatom, ein Halogenatom, einen Alkoxyrest, einen Aminorest oder einen durch wenigstens einen Alkylrest substituierten Aminorest bedeutet, $R^{19}$ ein Wasserstoffatom, einen Hydroxyrest oder einen Mercaptorest bedeutet, E ein Kohlenstoffatom oder ein Stickstoffatom bedeutet, und n eine ganze Zahl von 1 bis 4 ist, oder in welcher n gleich 2 ist und die beiden $R^{18}$ benachbart sind und zusammen einen aromatischen Ring bilden.

**20.** Verfahren gemäß einem der Ansprüche 1 bis 14, dadurch gekennzeichnet, daß der zweite Ligand ein Kondensationsprodukt der der Formel

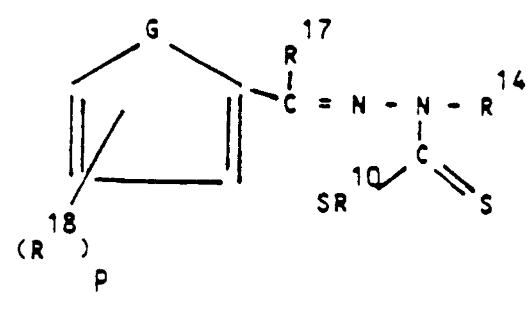

entsprechenden Dithiocarbazinsäure ist, in welcher $R^{10}$ ein Wasserstoffatom, einen Alkylrest oder einen Arylrest bedeutet; $R^{14}$ ein Wasserstoffatom, einen Alkylrest, einen Arylrest, einen Alkoxyrest, einen durch wenigstens eine aus den Hydroxy-, Carboxy-, Amino-, Amido- und Mercaptoresten ausgewählte Gruppierung substituierten Alkylrest oder einen durch mindestens eine aus den Halogenatomen und den Alkoxy-, Hydroxy-, Amino-, Mercapto- und wenigstens durch einen Alkylrest substituierten Aminoresten ausgewählte Gruppierung substituierten Arylrest bedeutet; $R^{17}$ ein Wasserstoffatom, einen Alkylrest, einen durch wenigstens eine aus den Hydroxy-, Carboxy-, Amino-, Amido- und Mercaptoresten ausgewählte Gruppierung substituierten Alkylrest bedeutet, $R^{18}$ ein Wasserstoffatom, ein Halogenatom, einen Alkoxyrest, einen Aminorest oder einen durch wenigstens einen Alkylrest substituierten Aminorest bedeutet, G S oder O ist und p 1, 2 oder 3 ist.

**21.** Verfahren gemäß einem der Ansprüche 1 bis 14, dadurch gekennzeichnet, daß der zweite Ligand Hydrazin oder ein Hydrazinderivat ist, welches der Formel

$R^{20}$-NH-NH-$R^{21}$

entspricht, in welcher $R^{20}$ ein Wasserstoffatom oder ein Alkylrest ist, und $R^{21}$ ein Wasserstoffatom oder ein aus den Resten der Formel

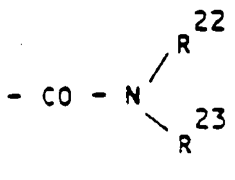
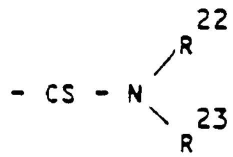
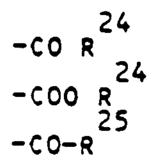
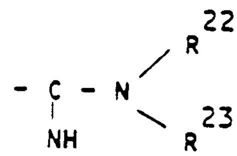

ausgewählter Rest ist, in welchen $R^{22}$ und $R^{23}$, welche identisch oder verschieden sein können, ein Wasserstoffatom, einen Alkylrest oder einen Aminorest bedeuten, $R^{24}$ einen Alkylrest bedeutet und $R^{25}$ einen nichtsubstituierten oder durch wenigstens eine aus den Halogenatomen und den Hydroxyresten ausgewählte Gruppierung substituierten Arylrest oder einen von einem nichtsubstituierten oder durch wenigstens eine aus den Halogenatomen und den Hydroxyresten ausgewählte Gruppierung substituierten Heterocyclus abgeleiteten Rest bedeutet.

**22.** Verfahren gemäß einem der Ansprüche 1 bis 14, dadurch gekennzeichnet, daß der zweite Ligand aus beta-N(2-Hydroxyphenyl)methylendithiocarbazinsäure-S-methylester, Dithiocarbazinsäure-S-methylester, N-Methyldithiocarbazinsäure-S-methylester, alpha-N-Methyl-beta-N-pyridylmethylendithiocarbazinsäure-S-methylester, beta-N-(2,5-Dihydroxyphenyl)-methylendithiocarbazinsäure-S-methylester, alpha-N-Methyl-beta-N(2-hydroxyphenyl)-methylendithiocarbazinsäure-S-methylester, Hydrazin, Semicarbazid, Thiosemicarbazid, 1-Methyl-3-thiosemicarbazid, 4-Methyl-3-thiosemicarbazid, Aminoacetonsemicarbazon, Carbohydrazid, Thiocarbohydrazid, Acethydrazid, Hydrazincarbonsäuremethylester, 2-Furoylhydrazid, Salicyloylhydrazid, Aminoguanidin und Natriumazid ausgewählt ist.

**23.** Kit zur Herstellung eines Technetium-nitridokomlexes durch Ausführung des Verfahrens gemäß einem der Ansprüche 1 bis 22, dadurch gekennzeichnet, daß er

- ein erstes, den ersten Liganden enthaltendes Gefäß und
- ein zweites, den zweiten Stickstoffliganden enthaltendes Gefäß

enthält.

**24.** Kit gemäß Anspruch 23, dadurch gekennzeichnet, daß er ein drittes Gefäß enthält, welches einen dritten organischen Liganden mit einer nukleophilen Gruppierung, einen monoklonalen Antikörper oder ein Antikörper-Fragment enthält.